# EUROPÄISCHE PATENTSCHRIFT

(11) **EP 1 472 224 B1**
(45) Veröffentlichungstag und Bekanntmachung des Hinweises auf die Patenterteilung: **11.07.2012**
(21) Anmeldenummer: 03706358.3
(22) Anmeldetag: 15.01.2003
(51) Int. Cl.: C07D 213/74, A61K 31/4402, A61P 17/00, A61P 11/00, A61P 9/00

(54) **3-ALKANOYLAMINO-PROPIONSÄURE-DERIVATE ALS INHIBITOREN DES INTEGRINS AVSS6**
3-ALKANOYLAMINO-PROPIONIC ACID DERIVATIVES USED AS INHIBITORS OF INTEGRIN AVSS6
3-ALKANOYLAMINO-ACIDE PROPIONIQUE DÉRIVÉS UTILISÉS COMME INHIBITEURS DE L'INTÉGRINE AVSS6

(30) Priorität: 06.02.2002 DE 10204789
(43) Veröffentlichungstag der Anmeldung: 03.11.2004
(73) Patentinhaber: Merck Patent GmbH, 64293 Darmstadt (DE)
(72) Erfinder: STÄHLE, Wolfgang, 55218 Ingelheim (DE); SCHADT, Oliver, 63517 Rodenbach (DE); JONCZYK, Alfred, 64295 Darmstadt (DE); GOODMAN, Simon, 64347 Griesheim (DE)
(86) Internationale Anmeldenummer: PCT/EP2003/000327
(87) Internationale Veröffentlichungsnummer: WO 2003/066594

(56) Entgegenhaltungen:
- WO-A-00/48996
- DE-A- 10 063 173

## Beschreibung

Die Erfindung betrifft neue Integrinantagonisten der Formel I 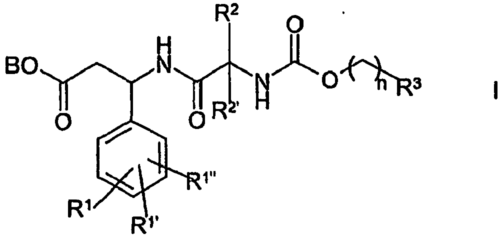 worin
- R¹, R^{1'}, R^{1"}: H, A, Ar, Het¹, Hal, NO₂, CN, OR⁴, COA, NHCOA, NH(CHO), NR⁴, COOR⁴ und/oder CONHR⁴₂
- R²: A, Ar, (CH₂)ₘXA, (CH₂)ₘOH, (CH₂)ₘNH₂, (CH₂)ₘNHA, (CH₂)ₘNA₂, (CH₂)ₘNHCOA, (CH₂)ₘNO₂, (CH₂)ₘCOOR¹, (CH₂)ₘCONH₂, (CH₂)ₘXCH₂)ₒAr, (CH₂)ₘX(CH₂)ₒCHAr₂, (CH₂)ₘX(CH₂)ₒCAr₃, (CH₂)ₘXCOYA, (CH₂)ₘXCOY(CH₂)ₒAr, (CH₂)ₘX(CH₂)ₒHet¹, (CH₂)ₘXCH₂)ₒCHHet¹₂, (CH₂)ₘX(CH₂)ₒCHet¹₃, (CH₂)ₘX(CH₂)ₒYA, (CH₂)ₘX(CH₂)ₒNHCOA, (CH₂)ₘNHCONHR^{2'}, (CH₂)mCH₂A, (CH₂)ₘCHA₂, (CH₂)ₘCA₃, (CH₂)ₘAr, (CH₂)ₘCHAr₂, (CH₂)ₘCAr₃, (CH₂)ₘHet¹, (CH₂)ₘCHHet¹₂, (CH₂)ₘCHet¹₃, (CH₂)ₘCycloalkyl, (CH₂)ₘ-NH-C(=NH)-NH₂, (CH₂)ₘ-(HN=)C-NH₂,
wobei X und Y unabhängig voneinander S, O, S=O, SO₂ oder NH sein können wobei wenn R² = (CH₂)ₘXCOYA oder (CH₂)ₘXCOY(CH₂)ₒAr ist, X und Y nicht S=O oder SO₂ sein können
- R^{2'}: H, A
- R² und R^{2'}: zusammen auch -(CH₂)ₚ- sein können
- R³: A-C(=NH)-NH-, Het²- oder Het²-NH-, wobei die primären Aminogruppen auch mit konventionellen Aminoschutzgruppen versehen sein können,
- R⁴: H, A, Het¹, Hal, NO₂, CN
- A: Alkyl mit 1 bis 8 C-Atomen
- B: H oder A
- Ar: unsubstituiertes oder ein- oder mehrfach durch Hal, A, OA, OH, CO-A, CN, COOA, COOH, CONH₂, CONHA, CONA₂, CF₃, OCF₃ oder NO₂ substituiertes Phenyl, Naphthyl, Anthranyl oder Biphenyl
- Het¹: einen aromatischen ein- oder zweikernigen Heterocyclus mit 1 bis 3 N-, O- und / oder S-Atomen, der unsubstituiert oder ein- oder zweifach durch F, Cl, Br, A, OA, SA, OCF₃, -CO-A, CN, COOA, CONH₂, CONHA, CONA₂, NA₂ oder NO₂ substituiert sein kann
- Het²: einen ein- oder zweikernigen Heterocyclus mit 1 bis 4 N-Atomen, der unsubstituiert oder ein- oder zweifach durch NH₂, NHA oder A substituiert sein kann,
- m: 0, 1, 2, 3, 4, 5, 6, oder 8
- n: 1, 2, 3, 4, 5 oder 6
- o: 0,1, 2 oder 3
- p: 2, 3, 4 oder 5
bedeutet,
deren Stereoisomere sowie deren physiologisch unbedenklichen Salze und Solvate.

Verbindungen mit teilweise ähnlicher Struktur sind offenbart in WO96/22966 A1, WO 97/08145 A1 und WO 00/48996 A2 wobei alle Verbindungen als Integrininhibitoren wirksam sind. Integrine sind membrangebundene, heterodimere Glycoproteine, die aus einer α-Untereinheit und einer kleineren β-Untereinheit bestehen. Die relative Affinität und Spezifität für eine Ligandenbindung wird durch Kombination der verschiedenen α- und β-Untereinheiten bestimmt. Gemäß der Offenbarung der genannten Patentanmeldungen hemmen die Verbindungen von WO 96/22966 A1 selektiv den α₄β₁-Integrinrezeptor und die Verbindungen von WO 97/08145 A1 selektiv den αᵥβ₃-Integrinrezeptor. Die Verbindungen von WO 00/48996 A2 hemmen vornehmlich αᵥβ₃ und αᵥβ₅-Integrinrezeptoren.

Der Erfindung lag die Aufgabe zugrunde, neue Verbindungen mit wertvollen Eigenschaften aufzufinden, insbesondere solche, die zur Herstellung von Arzneimitteln verwendet werden.

Es wurde gefunden, daß die Verbindungen der Formel I und ihre Salze bei guter Verträglichkeit sehr wertvolle pharmakologische Eigenschaften besitzen. Überraschenderweise hemmen die erfindungsgemäßen neuen Verbindungen bevorzugt den αᵥβ₆-Integrinrezeptor.

Den Integrinen kommen unterschiedliche physiologische und pathologische Funktionen zu, die im Einzelnen beispielsweise folgenden Übersichtsarbeiten entnommen werden kann: Integrins and signal transduction. Dedhar-S, Curr-Opin-Hematol. 1999 Jan; 6(1): 37-43, Integrins take partners: cross-talk between integrins and other membrane receptors. Porter-JC; Hogg-N, Trends-Cell Biol. 1998 Oct; 8(10): 390-6*,* Regulation of integrin-mediated adhesion during cell migration. Cox-EA; Huttenlocher-A, Microsc-Res-Tech. 1998 Dec 1; 43(5): 412-9*,* The role of integrins in the malignant phenotype of gliomas. Uhm-JH; Gladson-CL; Rao-JS, Front-Biosci. 1999 Feb 15; 4: D188-99*, oder* Sperm disintegrins, egg integrins, and other cell adhesion molecules of mammalian gamete plasma membrane interactions. Evans-JP Front-Biosci. 1999 Jan 15; 4: D114-31*.*

Eine wichtige Rolle kommt dabei den αᵥ-Integrinen zu, wie z.B in The role of alpha v-integrins in tumour progression and metastasis. Marshall-JF; Hart-IR Semin-Cancer-Biol. 1996 Jun; 7(3): 129-38 oder The role of alpha v-integrins during angiogenesis. Eliceiri-BP and Cheresh-DA Molecular Medicine 4: 741-750 (1998*)* zu finden ist.

Unter diesen Integrinen findet man auch αᵥβ₆ Epithelial integrins. Sheppard-D Bioessays. 1996 Aug; 18(8): 655-60 und die beiden Integrine αᵥβ₃ und αᵥβ₅, die bekannte Adhäsionsrezeptoren darstellen, deren biologische Bedeutung z.B. in J.A. Varner et al. Cell Adhesion and Communication 3, 367-374 (1995) und in J. Samanen et al. Curr. Pharmaceutical Design, 3, 545-584 (1997) referiert wurden.

αᵥβ₆ ist ein relativ seltenes Integrin (Busk et al., 1992 J. Biol. Chem. 267(9), 5790), das bei Reparaturvorgängen in Epithelgewebe vermehrt gebildet wird und die natürlichen Matrixmoleküle Fibronectin und Tenascin bevorzugt bindet (Wang et al., 1996, Am. J. Respir. Cell Mol. Biol. 15(5), 664). Daneben bindet auch Vitronectin an αᵥβ₆ *(*Characterization of the integrin alpha v beta 6 as a fibronectin-binding protein. Busk-M; Pytela-R; Sheppard-D. J-Biol-Chem. 1992 Mar 25; 267(9): 5790-6*;* Restricted distribution of integrin beta 6 mRNA in primate epithelial tissues. Breuss,-J-M; Gillett,-N; Lu,-L; Sheppard,-D; Pytela,-R J-Histochem-Cytochem. 1993 Oct; 41 (10): 1521-7*;* Differential regulation of airway epithelial integrins by growth factors. Wang-A; Yokosaki-Y; Ferrando-R; Balmes-J; Sheppard-D. Am-J-Respir-Cell-Mol-Biol. 1996 Nov; 15(5): 664-72*);* The integrin alphavbeta6 is critical for keratinocyte migration on both its known ligand, fibronectin, and on vitronectin. Huang,-X; Wu,-J; Spong,-S; Sheppard,-D J-Cell-Sci. 1998 Aug; 111 (Pt 15)2189-95*).*

Die physiologischen und pathologischen Funktionen von αᵥβ₆ sind noch nicht genau bekannt, es wird jedoch vermutet, daß dieses Integrin bei physiologischen Vorgängen und Erkrankungen (z. B. Entzündungen, Wundheilung, Tumore), bei denen epitheliale Zellen beteiligt sind, eine wichtige Rolle spielt (Expression of the beta 6 integrin subunit in development, neoplasia and tissue repair suggests a role in epithelial remodeling. Breuss,-J-M; Gallo,-J; DeLisser,-H-M; Klimanskaya,-I-V; Folkesson,-H-G; Pittet,-J-F; Nishimura,-S-L; Aldape,-K; Landers,-D-V; Carpenter,-W, et-al. J-Cell-Sci. 1995 Jun; 108 (Pt 6)2241-51*).*

So wird αᵥβ₆ auf Keratinozyten in Wunden exprimiert *(*Keratinocytes in human wounds express alpha v beta 6 integrin. Haapasalmi-K, Zhang-K, Tonnesen-M, Olerud-J, Sheppard-D, Salo-T, Kramer-R, Clark-RA, Uitto-VJ, Larjava-H. J-Invest-Dermatol. 1996 Jan, 106(1): 42-8*;* Epidermal integrin expression is upregulated rapidly in human fetal wound repair. Cass-D-L, Bullard-K-M, Sylvester-K-G, Yang-E-Y, Sheppard-D, Herlyn-M, Adzick-N-S J-Pediatr-Surg. 1998 Feb, 33(2): 312-6*)*, woraus anzunehmen ist, daß neben Wundheilungsprozessen und Entzündungen auch andere pathologische Ereignisse der Haut, wie z. B. Psoriasis, durch Agonisten oder Antagonisten des besagten Integrins beeinflußbar sind.

Weiterhin wird in Verhornungsstörungen der Haut (in der Mucosa der Mundhöhle, an den Lippen, der Zunge sowie den Genitalien), sogenannten Leukoplakien, αᵥβ₆ im Gegensatz zu normalem Vergleichsgewebe verstärkt exprimiert. Dabei nimmt die Häufigkeit und die Höhe der Expression von den Leukoplakien, über lichen planus, zum Plattenepithel-Karzinom (squamous cell carcinoma) zu, so dass ein Zusammenhang zwischen Expression von αᵥβ₆ und der malignen Transformation von Leukplakien zu vermuten ist: Expression of alpha(v)beta6 integrin in oral leukoplakia. Hamidi-S, Salo-T, Kainulainen-T, Epstein-J, Lemer-K, Larjava-H Br-J-Cancer. 2000 Apr, 82(8): 1433-40*;* Stromal fibroblasts influence oral squamous-cell carcinoma cell interactions with tenascin-C. Ramos-D-M, Chen-B-L, Boylen-K, Stern-M, Kramer-R-H, heppard D,Nishimura-S-L, Greenspan-D, Zardi-L, Pytela-R Int-J-Cancer. 1997 Jul 17, 72(2): 369-76*;* Expression of the alpha v beta 6 integrin promotes migration and invasion in squamous carcinoma cells Thomas-GJ, Lewis-MP, Whawell-SA, Russell-A, Sheppard-D, Hart-IR, Speight-PM, Marshall-JF JOURNAL-OF-INVESTIGATIVE-DERMATOLOGY. JUL 2001; 117 (1) : 67-73*;* Integrins alpha5beta1, alphavbeta1, and alphavbeta6 collaborate in squamous carcinoma cell spreading and migration on fibronectin. Koivisto,-L; Grenman-R, Heino-J, Larjava-H Exp-Cell-Res. 2000 Feb 25, 255(1): 10-7*).*

Ferner spielt αᵥβ₆ im Atemwegsepithel eine Rolle *(*Weinacker et al., 1995, Am. J. Respir. Cell Mol. Biol. 12(5), 547-56*;* Expression of the human integrin beta6 subunit in alveolar type 11 cells and bronchiolar epithelial cells reverses lung inflammation in beta6 knockout mice. Huang X, Wu J, Zhu W, Pytela R, Sheppard D, Am-J-Respir-Cell-Mol-Biol. 1998 Oct, 19(4): 636-42*;* Expression of integrin cell adhesion receptors during human airway epithelial repair in vivo. Pilewski JM, Latoche JD, Arcasoy SM, Albelda-S-M Am-J-Physiol. 1997 Jul, 273(1 Pt 1): L256-63*;* Global analysis of gene expression in pulmonary fibrosis reveals distinct programs regulating lung inflammation and fibrosis. Kaminski,-N; Allard JD, Pittet JF, Zuo F, Griffiths MJ, Morris D, Huang X, Sheppard D, Heller RA, Proc-Natl-Acad-Sci-U-S-A. 2000 Feb 15, 97(4): 1778-83)*,* so daß entsprechende Agonisten / Antagonisten dieses Integrins bei Atemwegserkrankungen, wie Bronchitis, Asthma, Lungenfibrosen und Atemwegstumoren erfolgreich eingesetzt werden könnten.

Neben der Lunge (Bronchien) können Fibrosen auch in anderen Organen auftreten, wie z.B. in der Haut, der Leber (bis zur Zirrhose), der Niere und der Blase, des Herzens und der Bauchspeicheldrüse (Mukoviszidose). Es ist zu vermuten, daß das Integrin αᵥβ₆ auch in diesen krankhaften Bindegewebsvermehrungen eine Rolle spielt und der Krankheitsverlauf daher durch Agonisten /Antagonisten des Integrins αᵥβ₆. beinflussbar ist *(*Mechanisms of tissue repair: from wound healing to fibrosis, Mutsaers SE, Bishop JE, Mcgrouther G, Laurent G, J Int. J. Biochem. Cell Biol. (1997) 29(1): 5-17*;* avb6 Integrin mediates latent TGFß activation: Implications for cutaneous fibrosis. Dalton SL, J.Am.Acad.Dermatol (1999) 41: 457-463*;* Clinical significance of blood serum connective tissue components in organ fibrosis, Kropf J, Gressner AM, Z. Med. Laboratoriumsdiagn. (1991) 32(3/4): 150-8*;* Angiotensin II, adhesion, and cardiac fibrosis, Schnee JM, Hsueh WA, Cardiovasc. Res. (2000) 46(2): 264-268*;* Pulmonary fibrosis and its treatment: today and in the next millennium. Sime P, J. Curr. Opin. Anti-Inflammatory Immunomodulatory Invest. Drugs (1999) 1(5): 423-432*;* Hepatic fibrosis: pathophysiology and laboratory diagnosis, Housset C, Guechot J, Pathol. Biol. (1999) 47(9): 886-894*;* Progressive renal disease. Fibroblasts, extracellular matrix, and integrins, Norman JT, Fine LG, Exp. Nephrol. (1999) 7(2): 167-177*;* Renal fibrosis: insights into pathogenesis and treatment, Nahas AM EI, Muchaneta-Kubara EC, Essawy M, Soylemezoglu O, Int. J. Biochem. Cell Biol. (1997) 29(1): 55-62*).*

Weiterhin ist bekannt, daß αᵥβ₆ auch im Darmepithel eine Rolle spielt, so daß entsprechende Integrin-Agonisten/-Antagonisten bei der Behandlung von Entzündungen, Tumoren und Wunden des Magen/Darmtraktes Verwendung finden könnten. Dabei gibt es Hinweise, dass das Integrin αᵥβ₆ auch die Sekretion von Matrix-Metalloproteasen beeinflusst, wie z.B. die der Gelatinase B (MMP-9): The alpha v beta 6 integrin promotes proliferation of colon carcinoma cells through a unique region of the beta 6 cytoplasmic domain, Agrez M, Chen A, Cone RI, Pytela R, Sheppard D, J Cell Biol (1994) 127(2): 547-56*;* Integrin-mediated signalling of gelatinase B secretion in colon cancer cells, Niu J, Gu X, Turton J, Meldrum C, Howard EW, Agrez M, Biochem Biophys Res Commun (1998) 249(1): 287-91.

Es wurde gezeigt, dass die Expression von αᵥβ₆ mit Veränderungen der Zelldichte und MMP-Aktivität einhergeht (The alpha v beta 6 integrin regulates its own expression with cell crowding: Implications for tumour progression, Niu J, Gu X, Ahmed N, Andrews S, Turton J, Bates R, Agrez M, INTERNATIONAL JOURNAL OF CANCER, (2001) 92 (1): 40-48*;* The alpha v beta 6 integrin induces gelatinase B secretion in colon cancer cells, Agrez M, Gu X, Turfon J, Meldrum C, Niu J, Antalis T, Howard EW, Int J Cancer (1999) 81(1): 90-7*;* alpha v beta 6 integrin upregulates matrix metalloproteinase 9 and promotes migration of normal oral keratinocytes, Thomas GJ, Poomsawat S, Lewis MP, Hart IR, Speight PM, Marshall JF, JOURNAL OF INVESTIGATIVE DERMATOLOGY (2001) 116 (6): 898-904*;* alpha V beta 6 integrin promotes invasion of squamous carcinoma cells through up-regulation of matrix metalloproteinase-9, Thomas GJ, Lewis MP, Hart IR, Marshall JF, Speight PM, INTERNATIONAL JOURNAL OF CANCER (2001) 92 (5): 641-650*).* Somit könnte die Regelung der MMP-Aktivität (möglicherweise verschiedener MMPs) durch Tumorzellen in Abhängigkeit ihrer Dichte ein Mechanismus sein, der den Zellen ermöglicht, beim Wachsen der Tumormasse, sich durch Proteolyse der umgebenden Matrix erneute Platz zu verschaffen - für Proliferation und Migration.

Aufgrund der Rolle des Integrin αᵥβ₆ bei Infektionsvorgängen ist zu vermuten, daß seine Agonisten/Antagonisten auch bei mikrobiellen Infektionen Verwendung finden können (Protozoen, Mikrophyten; Bakterien, Viren, Hefen, Pilze). Der Zusammenhang mit dem αᵥβ₆ Integrin ist beispielsweise für den Coxsackievirus beschrieben oder für den Befall von Wirtszellen mit dem Virus der Maul-und-Klauenseuche (FMDV), der αᵥβ₃ abhängig verlaufen, jedoch auch αᵥβ₆-abhängig erfolgen kann *(*Integrin alpha v beta 6 enhances coxsackievirus B1 lytic infection of human colon cancer cells. Agrez MV, Shafren DR, Gu X, Cox K, Sheppard D, Barry RD, Virology (1997) 239(1): 71-7*;* The epithelial integrin alphavbeta6 is a receptor for foot-and-mouth disease virus, Jackson T, Sheppard D, Denyer M, Blakemore W, King AM, J Virol (2000) 11: 4949-56*;* Role of the cytoplasmic domain of the beta-subunit of integrin alpha(v)beta6 in infection by foot-and-mouth disease virus, Miller LC, Blakemore W, Sheppard D, Atakilit A, King AM, Jackson T, J Virol (2001) 75(9): 4158-64*;* The ability of integrin avb3 to function as a receptor for foot-and-mouth disease virus is not dependent on the presence of complete subunit cytoplasmic domains, Neff S, Baxt B, J Virol (2001) 75(1): 527-532*;* Foot-and-mouth disease virus virulent for cattle utilizes the integrin avb3 as its receptor, Neff S, Sa-Carvalho D, Rieder E, Mason, PW, Blystone SD, Brown EJ, Baxt B, J Virol (1998) 72(5): 3587-3594*;* Arginine-glycine-aspartic acid-specific binding by foot-and-mouth disease viruses to the purified integrin avb3 in vitro, Jackson T, Sharma A, Ghazaleh RA, Blakemore WE, Ellard FM, Simmons DL, Newman JWI, Stuart DI, King AMQ, J Virol (1997) 71(11): 8357-8361*).*

Auch die Infektion mit *HIV* (AIDS) ist von αᵥβ Integrinen abhängig, so dass die Agonisten/Antagonisten des Integrine αᵥβ₆ hier ebenfalls eingesetzt werden könnten *(A novel integrin specificity for the human immunodeficiency virus (HIV) Tat protein, Ruoslahti EI, Vogel BE, Wong-Staal FY, PCT Int. Appl (1992)* WO 9214755*)*.

Nach neueren Erkenntnissen sekretiert das Bakterium Bacillus anthracis ein Toxin, dass aus 3 Proteinen besteht, von denen eines, das sogenannte PA oder Protective Antigen, an zellmembranständige Rezeptoren (Anthrax Toxin Receptor, ATR) bindet. ATR ist ein Typ I Membranprotein mit einer extrazellulären Domäne vom Typ des Willebrandt Factor (vWF A). Auch Integrine enthalten solche vWF A Domänen. Sowohl für Integrin αᵥβ₆ ist dies über eine Homologiebetrachtung in der Datenbank Swiss Prot nachvollziehbar (http://www.expasy.ch/cgi-bin/niceprot.pl?P18564; hier Sequenz β₆(131-371)), als auch für αᵥβ₃ (http://www.expasy.ch/cgibin/niceprot.pl?P05106; β₃ (135-377)). Es ist daher zu vermuten, dass αᵥβ₆ Agonisten/Antagonisten auch bei Anthrax (Milzbrand der Lunge, der Haut und des Darmes) nutzbar sind *(*Identification of the cellular receptor for anthrax toxin. K.A. Bradley et al. Nature 414, 225-229 (2001*) [and* accompanying articles]; Evolution of von Willebrand factor A (vWA) domains, Tuckwell D, Biochem Soc Trans (1999) 27(6): 835-840*).*

Aus der Abhängigkeit des Befalls von Wirtszellen von deren Adhäsionsrezeptoren für Bakterien sowie für Hefen (Sprosspilzen, Candida) *(*Cell adhesion molecules in the pathogenesis of and host defence against microbial infection, Kerr JR, Medical Microbiology, Manchester Royal Infirmary, UK, MOLECULAR PATHOLOGY (1999) 52(4): 220-30*;* Vitronectin-dependent invasion of epithelial cells by Neisseria gonorrhoeae involves alpha(v) integrin receptors, Dehio M, Gomez-Duarte OG, Dehio C, Meyer TF, FEBS LETTERS (1998) 424(1-2): 84-8*;* A natural variant of the cysteine protease virulence factor of group A Streptococcus with an arginine-glycine-aspartic acid (RGD) motif preferentially binds human integrins alphavbeta3 and alphaIIbbeta3, Stockbauer KE, Magoun L, Liu M, Burns EH Jr, Gubba S, Renish S, Pan X, Bodary SC, Baker E, Coburn J, Leong JM, Musser JM, PROCEEDINGS OF THE NATIONAL ACADEMY OF SCIENCES OF THE UNITED STATES OF AMERICA (1999), 96(1): 242-7*;* Involvement of alpha(v)beta3 integrinlike receptor and glycosaminoglycans in Candida albicans germ tube adhesion to vitronectin and to a human endothelial cell line, Santoni G, Spreghini E, Lucciarini R, Amantini C, Piccoli M, MICROBIAL-PATHOGENESIS (2001) 31(4): 159-72*)* ergibt sich die Verwendbarkeit von Agonisten/Antagonisten des Integrin αᵥβ₆ auch in diesen Fällen.

Das Integrin αᵥβ₆ tritt in Wechselwirkung mit TGF-β und führt dabei zu dessen Aktivierung (avb6 Integrin mediates latent TGFß activation: Implications for cutaneous fibrosis, Dalton SL, J Am Acad Dermatol (1999) 41: 457-463*;* The integrin avb6 binds and activates latent TGFb1: a mechanism for regulating pulmonary inflammation and fibrosis, Munger JS et al. Cell (1999) 96: 319-328*).* Latentes TGFβ₁ (eine der Pro-Formen) bindet an das Integrin αᵥβ₆ und wird hierdurch proteolytisch aktiviert. Die erfindungsgemäßen Agonisten/Antagonisten des Integrins αᵥβ₆ könnten somit über Inhibition der Bindung von TGF-β (Pro-Form, LAP-Peptid, LAP-TGFβ, Latentes TGF) die Aktivierung von TGF-β, und anderen Subtypen verhindern und hierdurch die Wirkung von TGFβ modulieren.

Bisher wurden 3 humane TGFβ-Isoformen entdeckt wurde, denen eine Rolle bei einer Vielzahl von Wachstums- und Differenzierungprozessen, insbesondere aber bei entzündlichen Prozessen, Fibrosen, Wundheilung, Knochenwachstum, der Modulation von Immunfunktionen, bei Angiogenese und Tumormetastase zugeordnet wird *(*Rifkin DB et al., Thrombosis and Haemostasis (1993) 70: 177-179*;* Hata A et al., Molecular Medicine Today (June 1998) 257-262*;* Integrin-mediated activation of transforming growth factor-beta(1) in pulmonary fibrosis, Sheppard DC, (2001) 120(1 Suppl): 49S-53S*;* Wickstom P et al., Prostate (1998) 37: 19-29*)*. Die erfindungsgemäßen Agonisten/Antagonisten von αᵥβ₆ könnten somit auch in diesen Prozessen einsetzbar sein.

Eine weitere Arbeit, die die Rolle von αᵥβ₆ bei immunologischen Vorgängen hervorhebt ist, beschreibt den Influx von Neutrophilen nach chemischer Schädigung der Lunge *(*Expression of the beta6 integrin subunit is associated with sites of neutrophil influx in lung epithelium, Miller LA, Barnett NL, Sheppard D, Hyde DM, J Histochem Cytochem (2001) 49(1): 41-8*).*

Die Wirkung einer Verbindung auf einen αᵥβ₆-Integrinrezeptor und damit die Aktivität als Inhibitor kann z.B. nach der Methode nachgewiesen werden, die von J.W. Smith et al. in J. Biol. Chem. 1990, 265, 12267-12271 beschrieben wird.

Neben der bevorzugten Hemmung von αᵥβ₆-Integrin-Rezeptoren wirken die Verbindungen auch als Inhibitoren der αᵥβ₃- oder αᵥβ₅-Integrin-Rezeptoren sowie als Inhibitoren des Glycoproteins IIb/IIIa. Das αᵥβ₃ Integrin beispielsweise wird auf einer Reihe von Zellen, z.B. Endothelzellen, Zellen der glatten Gefäßmuskulatur beispielsweise der Aorta, Zellen zum Abbau von Knochenmatrix (Osteoclasten) oder Tumorzellen, exprimiert.

Die Wirkung der erfindungsgemäßen Verbindungen auf die verschiedenen Integrin-Rezeptoren kann z.B. nach der Methode nachgewiesen werden, die von J.W. Smith et al. in J. Biol. Chem. 1990, 265, 12267-12271 beschrieben wird.
Die Abhängigkeit der Entstehung von Angiogenese von der Wechselwirkung zwischen vaskulären Integrinen und extrazellulären Matrixproteinen ist von P.C. Brooks, R.A. Clark und D.A. Cheresh in Science 1994, 264, 569-571 beschrieben.

Die Möglichkeit der Inhibierung dieser Wechselwirkung und damit zum Einleiten von Apoptose (programmierter Zelltod) angiogener vaskulärer Zellen durch ein cyclisches Peptid ist von P.C. Brooks, A.M. Montgomery, M. Rosenfeld, R.A. Reisfeld, T. Hu, G. Klier und D.A. Cheresh in Cell 1994, 79, 1157-1164 beschrieben. Es wurden darin z.B. αᵥβ₃-Antagonisten oder Antikörper gegen αᵥβ₃ beschrieben, die eine Schrumpfung von Tumoren durch Einleiten von Apoptose bewirken.

Der experimentelle Nachweis, daß auch die erfindungsgemäßen Verbindungen die Anheftung von lebenden Zellen auf den entsprechenden Matrixproteinen verhindern und dementsprechend auch die Anheftung von Tumorzellen an Matrixproteine verhindern, kann in einem Zelladhäsionstest erbracht werden, analog der Methode von F. Mitjans et al., J. Cell Science 1995, 108, 2825-2838.

Die Verbindungen der Formel I können die Bindung von Metalloproteinasen an Integrine hemmen und so verhindern, daß die Zellen die enzymatische Aktivität der Proteinase nutzen können. Ein Beispiel ist in der Hemmbarkeit der Bindung von MMP-2- (Matrix-Metallo-Proteinase-2-) an den Vitronektin-Rezeptor αᵥβ₃ durch ein Cyclo-RGD-Peptid zu finden, wie in P.C. Brooks et al., Cell 1996, 85, 683-693 beschrieben.

Verbindungen der Formel I, die die Wechselwirkung von Integrinrezeptoren und Liganden, wie z.B. von Fibrinogen an, den Fibrinogenrezeptor (Glycoprotein IIb/IIIa) blockieren, verhindern als Antagonisten die Ausbreitung von Tumorzellen durch Metastase und können daher als antimetastatisch wirkende Substanzen bei Operationen eingesetzt werden, bei denen Tumore chirurgisch entfernt oder angegriffen werden. Dies wird durch folgende Beobachtungen belegt:

Die Verbreitung von Tumorzellen von einem lokalen Tumor in das vaskuläre System erfolgt durch die Bildung von Mikroaggregaten (Mikrothromben) durch die Wechselwirkung der Tumorzellen mit Blutplättchen. Die Tumorzellen sind durch den Schutz im Mikroaggregat abgeschirmt und werden von den Zellen des Immunsystems nicht erkannt

Die Mikroaggregate können sich an Gefäßwandungen festsetzen, wodurch ein weiteres Eindringen von Tumorzellen in das Gewebe erleichtert wird. Da die Bildung der Mikrothromben durch Ligandenbindung an die entsprechenden Integrinrezeptoren, z.B. αᵥβ₃ oder α_{IIb}β₃, auf aktivierten Blutplättchen vermittelt wird, können die entsprechenden Antagonisten als wirksame Metastase-Hemmer angesehen werden.

Die Wirkung einer Verbindung auf einen αᵥβ₅-Integrinrezeptor und damit die Aktivität als Inhibitor kann z.B. nach der Methode nachgewiesen werden, die von J.W. Smith et al. in J. Biol. Chem. 1990, 265, 12267-12271 beschrieben wird.

Ein Maß für die Aufnahme eines Arzneimittelwirkstoffs in einen Organismus ist seine Bioverfügbarkeit.
Wird der Arzneimittelwirkstoff in Form einer Injektionslösung dem Organismus intravenös zugefügt, so liegt seine absolute Bioverfügbarkeit, d.h. der Anteil des Pharmakons, der unverändert im systemischen Blut, d.h. in den großen Kreislauf gelangt, bei 100%.
Bei oraler Vergabe eines therapeutischen Wirkstoffs liegt der Wirkstoff in der Regel als Feststoff in der Formulierung vor und muß sich daher zuerst auflösen, damit er die Eintrittsbarrieren, beispielsweise den Gastrointestinaltrakt, die Mundschleimhaut, nasale Membranen oder die Haut, insbesondere das Stratum corneum, überwinden kann bzw. vom Körper resorbiert werden kann. Daten zur Pharmakokinetik, d.h. zur Bioverfügbarkeit können analog zu der Methode von J. Shaffer et al, J. Pharm. Sciences, 1999, 88, 313-318 erhalten werden.
Ein weiteres Maß für die Resorbierbarkeit eines therapeutischen Wirkstoffes ist der logD-Wert, denn dieser Wert ist ein Maß für die Lipophilie eines Moleküls.

Die Verbindungen der Formel I besitzen mindestens ein chirales Zentrum und können daher in mehreren stereoisomeren Formen auftreten. Alle diese Formen (z.B. D- und L-Formen) und deren Gemische (z.B. die DL-Formen) sind in der Formel eingeschlossen.

Die erfindungsgemäßen Verbindungen nach Anspruch 1 können auch als sogenannte Prodrug-Derivate, synthetisiert werden, d.h. mit z.B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Ferner können freie Aminogruppen oder freie Hydroxygruppen als Substituenten von Verbindungen der Formel I mit entsprechenden Schutzgruppen versehen sein.

Unter Solvaten der Verbindungen der Formel I werden Anlagerungen von inerten Lösungsmittelmolekülen an die Verbindungen der Formel I verstanden, die sich aufgrund ihrer gegenseitigen Anziehungskraft ausbilden. Solvate sind z.B. Mono- oder Dihydrate oder Additionsverbindungen mit Alkoholen, wie z.B. mit Methanol oder Ethanol.

Gegenstand der Erfindung sind die Verbindungen der Formel I und ihre Salze und Solvate sowie ein Verfahren zur Herstellung von Verbindungen der Formel I sowie ihrer Salze und Solvate, dadurch gekennzeichnet, daß man
(a) eine Verbindung der Formel II 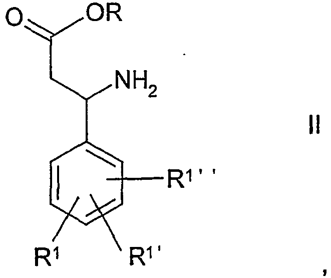 worin R eine Schutzgruppe ist und R¹, R^{1'}, R^{1"} die in Formel I angegebenen Bedeutungen haben und worin für den Fall dass R¹, R^{1'} und/oder R^{1"} freie Hydroxyl- und/oder Aminogruppen aufweisen diese jeweils durch eine Schutzgruppe geschützt vorliegen
   mit einer Verbindung der Formel III 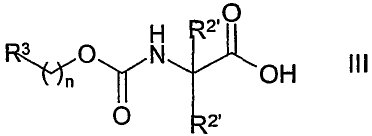 worin R¹, R^{2'}, R³ und n die in Formel I angegebenen Bedeutungen haben und worin für den Fall, dass R², R^{2'} und/oder R³ freie Hydroxyl- oder Aminogruppen enthalten, diese jeweils durch Schutzgruppen geschützt vorliegen
   umsetzt
   und die Schutzgruppe R sowie gegebenenfalls die an R¹, R^{1'}, R^{1"},
   R², R^{2'} und/oder R³ enthaltenen Schutzgruppen abspaltet,
   oder
(b) eine Verbindung der Formel IV 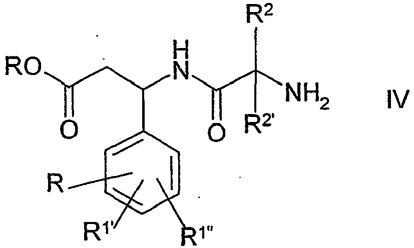 worin R eine Schutzgruppe ist und R¹, R^{1'}, R^{1"}, R² und R^{2'} die in Formel I angegebenen Bedeutungen haben und worin für den Fall, dass R¹, R^{1'}, R^{1"}, R² und/oder R^{2'} freie Hydroxyl- und/oder Aminogruppen enthalten, diese jeweils durch Schutzgruppen geschützt vorliegen
   mit einer Verbindung der Formel V 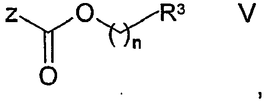 worin Z eine Abgangsgruppe wie z.B. Halogen oder eine reaktionsfähige veresterte OH-Gruppe bedeutet und n und R³ die in Formel I angegebenen Bedeutungen haben und worin für den Fall, dass R¹, R^{1'} und/oder R^{1"} freie Hydroxyl- und/oder Aminogruppen enthalten, diese jeweils durch Schutzgruppen geschützt vorliegen, umsetzt
   und die Schutzgruppe R sowie gegebenenfalls die an R¹, R^{1'}, R^{1"}, R², R^{2'} und/oder R³ enthaltenen Schutzgruppen abspaltet,
   oder
(c) in einer Verbindung der Formel I einen oder mehrere Reste R¹, R^{1'}, R^{1"}, R², R^{2'} und/oder R³ in einen oder mehrere Reste R¹, R^{1'}, R^{1"}, R², R^{2'} und/oder R³ umwandelt,
   indem man beispielsweise
   i) eine Hydroxygruppe alkyliert,
   ii) eine Estergruppe zu einer Carboxygruppe hydrolysiert,
   iii) eine Carboxygruppe verestert,
   iv) eine Aminogruppe alkyliert,
   v) ein Arylbromid oder -iodid durch eine Suzuki-Kupplung mit Boronsäuren zu den entsprechenden Kupplungsprodukten umsetzt, oder
   vi) eine Aminogruppe acyliert,
      oder
(d) eine Verbindung der Formel II mit einer Verbindung der Formel VI 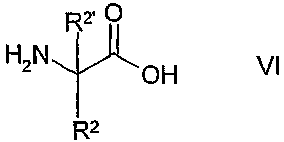 worin R² und R^{2'} die in Formel I angegebenen Bedeutungen haben und worin für den Fall, dass R² und/oder R^{2'} freie Hydroxyl- und/oder Aminogruppen enthalten, diese durch Schutzgruppen geschützt vorliegen,
   zu einer Verbindung der Formel IV umsetzt,
   die erhaltene Verbindung der Formel IV gemäß (b) mit einer Verbindung der Formel V zu einer Verbindung der Formel I umsetzt und anschließend die Schutzgruppe R sowie gegebenenfalls die an R¹, R^{1'}, R^{1"}, R², R^{2'} und/oder R³ enthaltenen Schutzgruppen abspaltet,
und/oder
eine basische oder saure Verbindung der Formel I durch Behandeln mit einer Säure oder Base in eines ihrer Salze oder Solvate umwandelt.

Für die gesamte Erfindung gilt, daß sämtliche Reste, die mehrfach auftreten, wie z.B. A, gleich oder verschieden sein können, d.h. unabhängig voneinander sind.

In den vorstehenden Formeln bedeutet A Alkyl, ist linear oder verzweigt, und hat 1 bis 8, vorzugsweise 1, 2, 3, 4, 5 oder 6 C-Atome. A bedeutet vorzugsweise Methyl, weiterhin Ethyl, n-Propyl, Isopropyl, n-Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, Heptyl oder Octyl. Weiterhin bevorzugte Ausführungsformen von A sind die genannten Alkylgruppen, die jedoch ein- oder mehrfach durch Hal oder NO₂ substituiert sein können, vorzugsweise Trifluormethyl, 2,2,2-Trifluorethyl oder 2-Nitroethyl, oder Alkylgruppen, deren Kohlenstoffkette durch -O- unterbrochen sein können, vorzugsweise -CH₂-O-CH₃, -CH₂-O-CH₂-CH₃ oder -CH₂-CH₂-O-CH₃-Besonders bevorzugt für A ist Methyl oder Ethyl.

R³ ist vorzugsweise z.B. Pyrimidin-2-ylamino, Pyridin-2-ylamino, Imidazol-1-yl, Imidazol-2-ylamino, Benzimidazol-2-ylamino, 4,5-Dihydro-imidazol-2-ylamino, 2-Amino-imidazol-5-ylamino, 2-Amino-pyridin-6-ylamino, 2-Amino-imidazol-5-yl oder 2-Amino-pyridin-6-yl.
R¹ ist vorzugsweise z.B. Phenyl.
Ar ist unsubstituiertes, vorzugsweise - wie angegeben - monosubstituiertes Phenyl, im einzelnen bevorzugt Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Cyanphenyl, o-, m- oder p-Methoxyphenyl, o-, m- oder p-Ethoxyphenyl, o-, m- oder p-Fluorphenyl, o-, m- oder p-Brornphenyl, o-, m- oder p- Chlorphenyl, o-, m- oder p-Methylthiophenyl, o-, m- oder p-Methylsulfinylphenyl, o-, m- oder p-Methylsulfonylphenyl, o-, m- oder p-Aminophenyl, o-, m- oder p-Methylaminophenyl, o-, m- oder p-Dimethylaminophenyl, o-, m- oder p-Nitrophenyl, o-, m- oder p-Acetylphenyl, o-, m- oder p-Methoxycarbonylphenyl, o-, m- oder p-Aminocarbonylphenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2-Chlor-3-methyl-, 2-Chlor-4-methyl-, 2-Chlor-5-methyl-, 2-Chlor-6-methyl-, 2-Methyl-3-chlor-, 2-Methyl-4-chlor-, 2-Methyl-5-chlor-, 2-Methyl-6-chlor-, 3-Chlor-4-methyl-, 3-Chlor-5-methyl- oder 3-Methyl-4-chlorphenyl, 2-Brom-3-methyl-, 2-Brom-4-methyl-, 2-Brom-5-methyl-, 2-Brom-6-methyl-, 2-Methyl-3-brom-, 2-Methyl-4-brom-, 2-Methyl-5-brom-, 2-Methyl-6-brom-, 3-Brom-4-methyl-, 3-Brom-5-methyl- oder 3-Methyl-4-bromphenyl, 2,4- oder 2,5-Dinitrophenyl, 2,5- oder 3,4-Dimethoxyphenyl, 2,3,4-, 2,3,5-, 2,3,6-, 2,4,6- oder 3,4,5-Trichlorphenyl, 2,4,6-Tri-tert.-Butylphenyl, 2,5-Dimethylphenyl, p-lodphenyl, 4-Fluor-3-chlorphenyl, 4-Fluor-3,5-dimethylphenyl, 2-Fluor-4-bromphenyl, 2,5-Difluor-4-bromphenyl, 2,4-Dichlor-5-methylphenyl, 3-Brom-6-methoxyphenyl, 3-Chlor-6-methoxyphenyl, 2-Methoxy-5-methylphenyl, 2,4,6-Triisopropylphenyl.

Cycloalkyl mit 3 bis 15 C-Atomen bedeutet vorzugsweise Cyclopropyl, Cyclobutyl, Cyclopentyl, Cyclohexyl, Cycloheptyl oder Cyclooctyl, besonders bevorzugt Cyclohexyl. Cycloalkyl bedeutet ebenfalls mono- oder bicyclische Terpene, vorzugsweise p-Menthan, Menthol, Pinan, Bornan oder Campher, wobei jede bekannte stereoisomere Form eingeschlossen ist oder Adamantyl. Für Campher bedeutet dies sowohl L-Campher als auch D-Campher. Besonders bevorzugt ist Cycloalkyl.

Hal bedeutet vorzugsweise F, Cl, Br oder Jod. Besonders bevorzugt ist Hal F oder Cl.

Aminoschutzgruppe bedeutet vorzugsweise Formyl, Acetyl, Propionyl, Butyryl, Phenylacetyl, Benzoyl, Toluyl, POA, Methoxycarbonyl, Ethoxycarbonyl, 2,2,2-Trichlorethoxycarbonyl, BOC, 2-lodethoxycarbonyl, CBZ ("Carbobenzoxy"), 4-Methoxybenzyloxycarbonyl, FMOC, Mtr oder Benzyl.

Het¹ ist vorzugsweise unsubstituiertes oder einfach durch F, Cl, Br, A, OA oder OCF₃ substituiertes 2,3-, 2,4- 2,5- oder 3,4-Thienyl, 2,3-, 2,4-, 2,5-oder 3,4-Pyrrolyl, 2,4-, 2,5- oder 4,5-Imidazolyl, 2,3-, 2,4-, 2,6- oder 3,5-Pyridyl, 2,4-, 2,5-, 2,6-, 4,5- oder 5,6-Pyrimidinyl, 1-, 2-, 3-, 4-, 5-, 6- oder 7-Indolyl. Besonders bevorzugt ist Pyridylamino.

Het² ist vorzugsweise unsubstituiertes oder ein- oder zweifach durch A, NHA und/oder NH₂ substituiertes 1-, 2- oder 3-Pyrrolyl, 1-, 2, 4- oder 5-Imidazolyl, 1-, 3-, 4- oder 5-Pyrazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Thiazol-1-, -3- oder 5-yl, 1- oder 5-Tetrazolyl, 3- oder 4-Pyridazinyl, Pyrazinyl, 1-, 2-, 3-, 4-, 5-, 6- oder 7-Indolyl, 1-, 2-, 4- oder 5-Benzimidazolyl, 1-, 3-, 4-, 5-, 6- oder 7-Benzopyrazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 1H-Imidazo[4,5-b]pyridin-2-yl oder 1,8-Naphthyridin-7-yl. Besonders bevorzugt ist 4-Pyridyl.

Die heterocyclischen Reste können auch teilweise oder vollständig hydriert sein. Het² kann also z. B. auch bedeuten 2,3-Dihydro-1-, -2-, -3-, -4- oder - 5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 4,5-Dihydro-imidazol-2-yl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, Morpholinyl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder-8-isochinolyl oder 1,2,3,4-Tetrahydro-1,8-naphthyridin-7-yl.
Hydrierte oder teilhydrierte Het²-Reste können zusätzlich durch =NH oder Carbonylsauerstoff substituiert sein.

n bedeutet vorzugsweise 2, 3, 4, 5 oder 6, ganz besonders bevorzugt bedeutet n 2, 3 oder 4.
m bedeutet vorzugsweise 0, 1, 2, 3, oder 4, ganz besonders bevorzugt bedeutet m 0, 1 oder 2.
o bedeutet vorzugsweise 0, 1 oder 2, ganz besonders bevorzugt bedeutet o1.

"mehrfach" substituiert bedeutet ein-, zwei-, drei- oder vierfach substituiert.

Pol bedeutet eine feste Phase ohne endständige funktionelle Gruppe, wie nachstehend näher erläutert. Der Begriff feste Phase und Harz wird im folgenden synonym verwendet.

Soweit die Verbindungen der Formel I Biphenyl enthalten, ist der zweite Phenylrest vorzugsweise in der 3- oder 4-Position an den ersten Phenylrest gekuppelt, besonders bevorzugt an die 4-Positon des ersten Phenylrings.

Dementsprechend sind Gegenstand der Erfindung insbesondere diejenigen Verbindungen der Formel I, in denen mindestens einer der genannte Reste eine der vorstehend angegebenen bevorzugten Bedeutungen hat. Einige bevorzugte Gruppen von Verbindungen können durch die folgenden Teilformeln Ia bis Ic ausgedrückt werden, die der Formel entspreche und worin die nicht näher bezeichneten Reste die bei der Formel I angegebene Bedeutung haben, worin jedoch.

| | |
|---|---|
| in Ia) R³ | Pyrimidin-2-ylamino, Pyridin-2-ylamino, Imidazol-1-yl, Imidazol-2-ylamino, Benzimidazol-2-ylamino, 4,5-Dihydro-ylamino, 2-Amino-pyridin-6-ylamino, 2-Amino-imidazol-5-yl oder 2-Amino-pyridin-6-yl bedeutet; |
| in Ib) R³ | Het²NH bedeutet, |
| in Ic) R³ | Het²NH bedeutet, worin |
| Het² | einen 5- oder 6-gliedrigen aromatischen oder gesättigten Heterocyclus mit 1 bis 2 N und/oder O-Atomen bedeutet; |
| in Id) R³ | Het²NH bedeutet, worin |
| Het² | pyridyl bedeutet; |
| in Ie) R³ | Het²NH |
| | worin |
| Het² | pyridyl bedeutet |
| in If) R¹, R^{1'}, R^{1"} | H, Ar, Het¹ Hal, NR⁴ und/oder CONHR⁴₂ bedeutet |
| | worin |
| R⁴ | H, A, Het¹ bedeutet |
| in Ig) R¹ | Ar |
| in Ih) R¹ | Ar |
| | worin |
| Ar | einen unsubstituierten oder ein- oder zweifach durch A, OA, OH, Hal, CF₃ substituierten Phenylrest bedeutet |
| R^{1'}, R^{1"} | jeweils H bedeutet |
| in Ii) R¹ | Ar |
| | worin |
| Ar | Phenyl |
| R^{1'}, R^{1"} | jeweils H bedeutet |
| in ij) R³ | Het²NH bedeutet, |
| | worin |
| Het² | pyridyl bedeutet, |
| R¹ | Ar |
| | worin |
| Ar | einen unsubstituierten oder ein- oder zweifach durch A, OA, OH, Hal, CF₃ substituierten Phenylrest bedeutet, |
| n | 2, 3, oder 4 bedeutet; |
| in Ik) R² | A, Ar, (CH₂)ₘXA, (CH₂)ₘOH, (CH₂)ₘNH₂, (CH₂)ₘNHA, (CH₂)ₘNA₂, (CH₂)ₘNHCOA (CH₂)ₘNO₂, (CH₂)ₘCOOR¹, (CH₂)ₘCONH₂, (CH₂)ₘX(CH₂)ₒAr, (CH₂)ₘX(CH₂)ₒCHAr₂, (CH₂)ₘX(CH₂)ₒCAr₃, (CH₂)ₘXCOYA, (CH₂)ₘXCOY(CH₂)ₒAr, (CH₂)ₘX(CH₂)ₒHet¹, (CH₂)ₘX(CH₂)ₒCHHet¹₂, (CH₂)ₘX(CH₂)ₒCHet¹₃, (CH₂)ₘX(CH₂)ₒYA, (CH₂)ₘX(CH₂)ₒNHCOA, (CH₂)ₘNHCONHR^{2'}, (CH₂)ₘAr, (CH₂)ₘCHAr₂, (CH₂)ₘCAr₃, (CH₂)ₘHet¹, (CH₂)ₘCHHet¹₂, (CH₂)ₘCHet¹₃, (CH₂)ₘCycloalkyl, (CH₂)ₘ-NH-C(=NH)-NH₂, (CH₂)ₘ-(HN=)C-NH₂, |
| | worin |
| X und Y | unabhängig voneinander S, O, S=O, SO₂ oder NH sein können, wobei wenn R² = (CH₂)ₘXCOYA oder |
| | (CH₂)ₘXCOY(CH₂)ₒAr ist, X und Y nicht S=O oder SO₂ sein können; |
| in Il) R² | A, Ar, (CH₂)ₘXA, (CH₂)ₘOH, (CH₂)ₘNH₂, (CH₂)ₘNHA, (CH₂)ₘNA₂, (CH₂)ₘNO₂, (CH₂)ₘCOOR¹, (CH₂)ₘCONH₂, (CH₂)ₘX(CH₂)ₒAr, (CH₂)ₘX(CH₂)ₒCHAr₂, (CH₂)ₘX(CH₂)ₒCAr₃, (CH₂)ₘXCOYA, (CH₂)ₘXCOY(CH₂)ₒAr, (CH₂)ₘX(CH₂)ₒHet¹, (CH₂)ₘX(CH₂)ₒCHHet¹₂, (CH₂)ₘX(CH₂)ₒCHet¹₃, (CH₂)ₘX(CH₂)ₒYA, (CH₂)ₘX(CH₂)ₒNHCOA, (CH₂)ₘNHCONHR^{2'}, (CH₂)ₘAr, (CH₂)ₘCHAr₂, (CH₂)ₘCAr₃, (CH₂)ₘHet¹, (CH₂)ₘCHHet¹₂, (CH₂)ₘCHet¹₃, (CH₂)ₘCycloalkyl, (CH₂)ₘ-NH-C(=NH)-NH₂, (CH₂)ₘ(HN=)C-NH₂, |
| | worin |
| R^{2'} | H |
| X und Y | unabhängig voneinander S, O, S=O, SO₂ oder NH sein können, |
| | wobei wenn R² = (CH₂)ₘXCOYA oder (CH₂)ₘXCOY(CH₂)ₒAr ist, X und Y nicht S=O oder SO₂ sein können, |
| m | 1, 2, 3 oder 4 bedeutet; |
| in Im) R² | A, Ar, (CH₂)ₘXA, (CH₂)ₘOH, (CH₂)ₘNH₂, (CH₂)ₘNHA, (CH₂)ₘNA₂, (CH₂)ₘNO₂, (CH₂)ₘCOOR¹, (CH₂)ₘCONH₂, (CH₂)ₘX(CH₂)ₒAr, (CH₂)ₘX(CH₂)ₒCHAr₂, (CH₂)ₘX(CH₂)ₒCAr₃, (CH₂)ₘXCOYA, (CH₂)ₘXCOY(CH₂)ₒAr, (CH₂)ₘX(CH₂)ₒHet¹, |
| | (CH₂)ₘX(CH₂)ₒCHHet¹₂, (CH₂)ₘX(CH₂)ₒCHet¹₃, (CH₂)ₘX(CH₂)ₒYA, (CH₂)ₘX(CH₂)ₒNHCOA, (CH₂)ₘNHCONHR^{2'}, (CH₂)ₘAr, (CH₂)ₘCHAr₂, (CH₂)ₘCAr₃, (CH₂)ₘHet¹, (CH₂)ₘCHHet¹₂, (CH₂)ₘCHet¹₃, (CH₂)ₘCycloalkyl, (CH₂)ₘ-NH-C(=NH)-NH₂, (CH₂)ₘ-(HN=)C-NH₂, |
| | worin |
| X und Y | unabhängig voneinander S, O, S=O, SO₂ oder NH sein können, |
| | wobei wenn R² = (CH₂)ₘXCOYA oder (CH₂)ₘXCOY(CH₂)ₒAr ist, X und Y nicht S=O oder SO₂ sein können |
| | und für den Fall, dass X und Y direkt durch eine chemische Bindung miteinander verbunden sind, diese jeweils S sind; |
| R^{2'} | H |
| m | 1, 2, 3 oder 4 |
| o | 0, 1, 2 oder 3 bedeutet; |
| in In) R² | A, Ar, (CH₂)ₘXA, (CH₂)ₘOH, (CH₂)ₘNH₂, (CH₂)ₘNHA, (CH₂)ₘNA₂, (CH₂)ₘNO₂, (CH₂)ₘCOOR¹, (CH₂)ₘCONH₂, (CH₂)ₘX(CH₂)ₒAr, (CH₂)ₘX(CH₂)ₒCHAr₂, (CH₂)ₘX(CH₂)ₒCAr₃, (CH₂)ₘXCOYA, (CH₂)ₘXCOY(CH₂)ₒAr, (CH₂)ₙX(CH₂)ₒHet¹, (CH₂)ₘX(CH₂)ₒCHHet¹₂, (CH₂)ₘX(CH₂)ₒCHet¹₃, (CH₂)ₘX(CH₂)ₒYA, (CH₂)ₘX(CH₂)ₒNHCOA, (CH₂)ₘNHCONHR^{2'}, (CH₂)ₘAr, (CH₂)ₘCHAr₂, (CH₂)ₘCAr₃, (CH₂)ₘHet¹, (CH₂)ₘCHHet¹₂, (CH₂)ₘCHet¹₃, (CH₂)ₘCycloalkyl, (CH₂)ₘNH- |
| | C(=NH)-NH₂, (CH₂)ₘ-(HN=)C-NH₂, |
| | worin |
| R^{2'} | H |
| Het¹ | einen ein- oder zweikernigen 5- und/oder 6-gliedrigen aromatischen oder gesättigten Heterocyclus mit 1 bis 2 N, S und/oder O-Atomen bedeutet, |
| Ar | einen unsubstituierten oder ein- oder zweifach durch A, OA, OH, Hal, CF₃ substituierten Phenylrest bedeutet, |
| X und Y | unabhängig voneinander S, O, S=O, SO₂ oder NH sein können, |
| | wobei wenn R² = (CH₂)ₘXCOYA oder (CH₂)ₘXCOY(CH₂)ₒAr ist, X und Y unabhängig voneinander NH und/oder O sind, |
| | und wobei für den Fall, dass X und Y direkt durch eine chemische Bindung miteinander verbunden sind, diese jeweils S sind; |
| m | 1, 2, 3 oder 4 |
| o | 0, 1, 2 oder 3 bedeutet; |
| in Io) R² | A, Ar, (CH₂)ₘXA, (CH₂)ₘOH, (CH₂)ₘNH₂, (CH₂)ₘNHA, (CH₂)ₘNA₂, (CH₂)ₘNO₂, (CH₂)ₘCOOR¹, (CH₂)ₘCONH₂, (CH₂)ₘX(CH₂)ₒAr, (CH₂)ₘX(CH₂)ₒCHAr₂, (CH₂)ₘX(CH₂)ₒCAr₃, (CH₂)ₘXCOYA, (CH₂)ₘXCOY(CH₂)ₒAr, (CH₂)ₘX(CH₂)ₒHet¹, (CH₂)ₘX(CH₂)ₒCHHet¹₂, (CH₂)ₘX(CH₂)ₒCHet¹₃, (CH₂)ₘX(CH₂)ₒYA, (CH₂)ₘX(CH₂)ₒNHCOA, (CH₂)ₘNHCONR^{2'}, (CH₂)ₘAr, (CH₂)ₘCHAr₂, |
| | (CH₂)ₘCAr₃, (CH₂)ₘHet¹, (CH₂)ₘCHHet¹₂, (CH₂)ₘCHet¹₃, (CH₂)ₘCycloalkyl, (CH₂)ₘ-NH-C(=NH)-NH₂, (CH₂)ₘ-(HN=)C-NH₂, |
| | worin |
| R^{2'} | H |
| Het¹ | Imidazolyl-, Thiophenyl-, Pyridinyl- oder Indolyl |
| Ar | Phenyl oder 4-OH-Phenyl bedeutet |
| X und Y | unabhängig voneinander S, O, S=O, SO₂ oder NH sein können, |
| | wobei wenn R² = (CH₂)ₘXCOYA oder (CH₂)ₘXCOY(CH_{Z})ₒAr ist, X = NH und Y = O ist und |
| | wobei für den Fall, dass X und Y direkt durch eine chemische Bindung miteinander verbunden sind, diese jeweils S sind; |
| m | 1, 2, 3 oder 4 |
| o | 0, 1, 2 oder 3 bedeutet; |
| in Ip) R³ | Het²NH bedeutet, |
| R¹, R^{1'}, R^{1"} | H, Ar, Het¹ Hal, NR⁴ und/oder CONHR⁴₂ bedeutet |
| | worin |
| R⁴ | H, A, Het¹ bedeutet |
| R² | A, Ar, (CH₂)ₘXA, (CH₂)ₘOH, (CH₂)ₘNH₂, (CH₂)ₘNHA, (CH₂)ₘNA₂, (CH₂)ₘNO₂, (CH₂)ₘCOOR¹, (CH₂)ₘCONH₂, |
| | (CH₂)ₘX(CH₂)ₒAr, (CH₂)ₘCH₂)ₒCHAr₂, (CH₂)ₘX(CH₂)ₒCAr₃, (CH₂)ₘXCOYA, (CH₂)ₘXCOY(CH₂)ₒAr, (CH₂)ₘX(CH₂)ₒHet¹, (CH₂)ₘX(CH₂)ₒCHHet¹₂, (CH₂)ₘX(CH₂)ₒCHet¹₃, (CH₂)ₘX(CH₂)ₒYA, (CH₂)ₘX(CH₂)ₒNHCOA, (CH₂NHCONR^{2'}, (CH₂)ₘAr, (CH₂)ₘCHAr₂, (CH₂)ₘCAr₃, (CH₂)ₘHet¹, (CH₂)ₘCHHet¹₂, (CH₂)ₘCHet¹₃, (CH₂)ₘCycloalkyl, (CH₂)ₘ-NH-C(=NH)-NH₂, (CH₂)ₘ-(HN=)C-NH₂, |
| | worin |
| X und Y | unabhängig voneinander S, O, S=O, SO₂ oder NH sein können, |
| | wobei wenn R² = (CH₂)ₘXCOYA oder (CH₂)ₘXCOY(CH₂)ₒAr ist, X und Y nicht S=O oder SO₂ sein können; |
| in Iq) R³ | Het²NH bedeutet, |
| | worin |
| Het² | einen 5- oder 6-gliedrigen aromatischen oder gesättigten Heterocyclus mit 1 bis 2 N und/oder O-Atomen bedeutet; |
| R¹, R^{1'}, R^{1"} | H, Ar, Het¹ Hal, NR⁴ und/oder CONHR⁴₂ bedeutet, worin R⁴ = H, A und/oder Het¹ ist und |
| | worin wenn R¹ = Ar bedeutet |
| R^{1'}, R^{1"} | jeweils H sind |
| | und |
| Ar , | einen unsubstituierten oder ein- oder zweifach durch A, OA, OH, Hal, CF₃ substituierten Phenylrest bedeutet |
| R² | A, Ar, (CH₂)ₘXA, (CH₂)ₘOH, (CH₂)ₘNH₂, (CH₂)ₘNHA, (CH₂)ₘNA₂, (CH₂)ₘNO₂, (CH₂)ₘCOOR¹, (CH₂)ₘCONH₂, (CH₂)ₘX(CH₂)ₒAr, (CH₂)ₘX(CH₂)ₒCHAr₂, (CH₂)ₘX(CH₂)ₒCAr₃, (CH₂)ₘXCOYA, (CH₂)ₘXCOY(CH₂)ₒAr, (CH₂)ₘX(CH₂)ₒHet¹, (CH₂)ₘX(CH₂)ₒCHHet¹₂, (CH₂)ₘX(CH₂)ₒCHet¹₃, (CH₂)ₘX(CH₂)ₒYA, (CH₂)ₘX(CH₂)ₒNHCOA, ((CH₂)ₘNHCONH₂, CH₂)ₘAr, (CH₂)ₘCHAr₂, (CH₂)ₘCAr₃, (CH₂)ₘHet¹, (CH₂)ₘCHHet¹₂, (CH₂)ₘCHet¹₃, (CH₂)ₘCycloalkyl, (CH₂)ₘ-NH-C(=NH)-NH₂, (CH₂)ₘ-(HN=)C-NH₂, |
| | worin |
| X und Y | unabhängig voneinander S, O, S=O, SO₂ oder NH sein können, |
| | wobei wenn R² = (CH₂)ₘXCOYA oder (CH₂)ₘXCOY(CH₂)ₒAr ist, X und Y nicht S=O oder SO₂ sein können, |
| m | 1, 2, 3 oder 4 bedeutet; |
| in Ir) R¹, R^{1'}, R^{1"} | H, Ar, Hal, NR⁴ und/oder CONHR⁴₂ bedeutet, worin R⁴ = H und/oder A ist und worin wenn R¹ = Ar bedeutet |
| R^{1'}, R^{1"} | jeweils H sind |
| | und |
| Ar | einen unsubstituierten oder ein- oder zweifach durch A, OA, OH, Hal, CF₃ substituierten Phenylrest bedeutet, |
| R³ | Het²NH bedeutet, |
| | worin |
| Het² | einen 5- oder 6-gliedrige aromatischen oder gesättigten Heterocyclus mit 1 bis 2 N und/oder O-Atomen bedeutet, |
| R² | A, Ar, (CH₂)ₘXA, (CH₂)ₘOH, (CH₂)NH₂, (CH₂)ₘNHA, (CH₂)ₘNA₂, (CH₂)ₘNO₂, (CH₂)ₘCOOR¹, (CH₂)ₘCONH₂, (CH₂)ₘX(CH₂)ₒAr, (CH₂)ₘX(CH₂)ₒCHAr₂, (CH₂)ₘX(CH₂)ₒCAr₃, (CH₂)ₘXCOYA, (CH₂)ₘXCOY(CH₂)ₒAr, (CH₂)ₘX(CH₂)ₒHet¹, (CH₂)ₘX(CH₂)ₒCHHet¹₂, (CH₂)ₘX(CH₂)ₒCHet¹₃, (CH₂)ₘX(CH₂)ₒYA, (CH₂)ₘX(CH₂)ₒNHCOA, (CH₂)ₘNHCONHR^{2'}, (CH₂)ₘAr, (CH₂)ₘCHAr₂, (CH₂)ₘCAr₃, (CH₂)ₘHet¹, (CH₂)ₘCHHet¹₂, (CH₂)ₘCHet¹₃, (CH₂)ₘCycloalkyl, (CH₂)ₘ-NH-C(=NH)-NH₂, (CH₂)ₘ-(HN=)C-NH₂, |
| | worin |
| R^{2'} 2- | H |
| Het¹ | Imidazolyl-, Thiophenyl-, Pyridinyl- oder Indolyl |
| Ar | Phenyl oder 4-OH-Phenyl bedeutet |
| X und Y | unabhängig voneinander S, O, S=O, SO₂ oder NH sein können, |
| | wobei wenn R² = (CH₂)ₘXCOYA oder (CH₂)ₘXCOY(CH_{z})ₒÄr ist, X und Y unabhängig voneinander NH und/oder O sind, |
| m | 1, 2, 3 oder 4 |
| o | 0, 1, 2 oder 3 bedeutet; |
| in Is) R³ | Het²NH |
| | worin |
| Het² | pyridyl bedeutet, |
| R¹, R¹¹, R^{1"} | H, Ar, und/oder Hal bedeutet, worin wenn R¹ = Ar bedeutet |
| R^{1'}, R^{1"} | jeweils H sind |
| | und |
| Ar | Phenyl bedeutet, |
| R² | A, Ar, (CH₂)ₘXA, (CH₂)ₘOH, (CH₂)ₘNH₂, (CH₂)ₘNHA, (CH₂)ₘNA₂, (CH₂)ₘNO₂, (CH₂)ₘCOOR¹, (CH₂)ₘCONH₂, (CH₂)ₘX(CH₂)ₒAr, (CH₂)ₘX(CH₂)ₒCHAr₂, (CH₂)ₘX(CH₂)ₒCAr₃, (CH₂)ₘXCOYA, (CH₂)ₘXCOY(CH₂)ₒAr, (CH₂)ₘX(CH₂)ₒHet¹, (CH₂)ₘX(CH₂)ₒCHHet¹₂, (CH₂)ₘX(CH₂)ₒCHet¹₃, (CH₂)ₘX(CH₂)ₒYA, (CH₂)ₘX(CH₂)ₒNHCOA, (CH₂)ₘNHCONH₂ (CH₂)ₘAr, (CH₂)ₘCHAr₂, (CH₂)ₘCAr₃, (CH₂)ₘHet¹, (CH₂)ₘCHHet¹₂, (CH₂)ₘCHet¹₃, (CH₂)ₘCycloalkyl, (CH₂)ₘ-NH-C(=NH)-NH₂, (CH₂)ₘ-(HN=)C-NH₂, |
| | worin |
| Het_{,} | Imidazolyl-, Thiophenyl-, Pyridinyl- oder Indolyl |
| Ar | Phenyl oder 4-OH-Phenyl bedeutet |
| X und Y | unabhängig voneinander S, O, S=O, SO₂ oder NH sein können, |
| | wobei wenn R² = (CH₂)ₘXCOYA oder (CH₂)ₘXCOY(CH₂)ₒAr ist, X und Y unabhängig voneinander NH und/oder O sind, und für den Fall, dass X und Y direkt durch eine chemische Bindung miteinander verbunden sind, diese jeweils S sind; |
| m | 1, 2, 3 oder 4 |
| o | 0, 1, 2 oder 3 bedeutet; |
| in It) R³ | Het²NH |
| | worin |
| Het² | pyridyl bedeutet, |
| R¹, R^{1'}, R^{1"} | H, Ar, und/oder Hal bedeutet, |
| | worin |
| Hal | F, Cl und/oder Br ist und worin wenn R¹ = Ar bedeutet |
| R^{1'}, R^{1"} | jeweils H sind |
| | und |
| Ar | Phenyl bedeutet, |
| R² | A, (CH₂)ₘXA, (CH₂)ₘOH, (CH₂)ₘNH₂, (CH₂)ₘNHA, (CH₂)ₘNA₂, (CH₂)ₘNO₂, (CH₂)ₘCOOH, (CH₂)ₘCONH₂, |
| | (CH₂)ₘX(CH₂)ₒAr, (CH₂)ₘX(CH₂)ₒCHAr₂, (CH₂)ₘX(CH₂)ₒCAr₃, (CH₂)ₘNHCOOA, (CH₂)ₘNHCOO(CH₂)ₒAr, (CH₂)ₘX(CH₂)ₒHet¹, (CH₂)ₘX(CH₂)ₒCHHet¹₂, (CH₂)ₘX(CH₂)ₒCHet¹₃, (CH₂)ₘX(CH₂)ₒYA, (CH₂)ₘX(CH₂)ₒNHCOA, (CH₂)ₘNHCONH₂ (CH₂)ₘAr, (CH₂)ₘCHAr₂, (CH₂)ₘCAr₃, (CH₂)ₘHet¹, (CH₂)ₘCHHet¹₂, (CH₂)ₘCHet¹₃, (CH₂)ₘCycloalkyl, (CH₂)ₘ-NH-C(=NH)-NH₂, (CH₂)ₘ-(HN=)C-NH₂, |
| R^{2'} | H, |
| R² und R^{2'} | zusammen auch -(CH₂)ₚ- sein können |
| Het¹ | Imidazolyl-, Thiophenyl-, Pyridinyl- oder Indolyl |
| Ar | Phenyl oder 4-OH-Phenyl |
| X | S, O, S=O, SO₂ oder N H |
| Y | S, 0 oder NH |
| m | 1, 2, 3 oder 4 |
| n | 2 oder 3 |
| o | 0 oder 1 |
| p | 5 bedeutet |
| | wobei wenn |
| X und Y | direkt durch eine chemische Bindung miteinander verbunden sind, diese jeweils S sind; |
| in Iu) R³ | Het²NH |
| | worin |
| Het² | pyridyl bedeutet, |
| R¹, R^{1'}, R^{1"} | H, Ar, und/oder Hal bedeutet, |
| | worin |
| Hal | F, Cl und/oder Br ist und worin wenn R¹ = Ar bedeutet |
| R^{1'}, R^{1"} | jeweils H sind |
| | und |
| Ar | Phenyl bedeutet, |
| R² | (CH₂)ₘX(CH₂)ₒAr, (CH₂)ₘX(CH₂)ₒCHAr₂ oder (CH₂)ₘX(CH₂)ₒCAr₃, |
| R^{2'} | H, |
| Ar | Phenyl oder 4-OH-Phenyl |
| X | S oder O |
| m | 1, 2, 3 oder 4 |
| n | 2 oder 3 |
| o | 0 oder 1 |
| | sind; |

Besonders bevorzugt sind die nachfolgend genannten Verbindungen der allgemeinen Formel I

3-Biphenyl-4-yl-3-{3-(1,1-diphenyl-methylsulfanyl)-2-[2-(pyridin-2-ylamino)-ethoxycarbonylamino]-propanoylamino}-propionsäure (EMD 393210) 3-Biphenyl-4-yl-3-{3-(1,1-diphenyl-methylsulfanyl)-2-[3-(pyridin-2-ylamino)-propoxycarbonylamino]-propanoylamino}- propionsäure (EMD 393215)
3-{3-Benzyloxy-2-[3-(pyridin-2-ylamino)-propoxycarbonylamino]-propanoylamino}-3-biphenyl-4-yl-propionsäure (EMD 393216) 3-{3-Benzyloxy-2-[2-(pyridin-2-ylamino)-ethoxycarbonylamino]-propanoylamino}-3-biphenyl-4-yl-propionsäure (EMD 393217) 3-{3-Benzyloxy-2-[2-(6-methylamino-pyridin-2-yl)-ethoxycarbonylamino]-propanoylamino}-3-biphenyl-4-yl-propionsäure (EMD 395936) 3-{3-Benzyloxy-2-[3-(pyridin-2-ylamino)-propoxycarbonylamino]-propanoylamino}-3-biphenyl-4-yl-propionsäure (EMD 397970) 3-{3-Benzyloxy-2-[2-(6-methyl-pyridin-2-ylamino)-ethoxycarbonylamino]-propanoylamino}-3-biphenyl-4-yl-propionsäure (EMD 408406) 3-{3-Benzyloxy-2-[3-(pyridin-2-ylamino)-propoxycarbonylamino]-propanoylamino}-3-(3,5-dichloro-phenyl)-propionsäureethylester (EMD 396493)
3-{3-Benzyloxy-2-[2-(6-methylamino-pyridin-2-yl)-ethoxycarbonylamino]-propanoylamino}-3-(3,5-dichloro-phenyl)-propionsäureethylester (EMD 396494)
3-{3-Benzyloxy-2-[2-(pyridin-2-ylamino)-ethoxycarbonylamino]-propanoylamino}-3-(3,5-dichloro-phenyl)-propionsäureethylester (EMD 396496)
3-{3-Benzyloxy-2-[3-(pyridin-2-ylamino)-propoxycarbonylamino]-propanoylamino}-3-(3,5-dichloro-phenyl)-propionsäure (EMD 397966) 3-{3-Benzyloxy-2-[2-(6-methyl-pyridin-2-ylamino)-ethoxycarbonylamino]-propanoylamino}-3-(3,5-dichloro-phenyl)-propionsäureethylester (EMD 408404)
3-{3-Benzyloxy-2-[2-(6-methyl-pyridin-2-ylamino)-ethoxycarbonylamino]-propanoylamino}-3-(3,5-dichloro-phenyl)- propionsäure (EMD 408407)
deren Stereoisomere sowie deren physiologisch unbedenklichen Salze und Solvate.

Die Verbindungen der Formel I nach Anspruch 1 und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I nach Anspruch 1 umsetzt.

Es können auch mehrere - gleiche oder verschiedene - geschützte Amino-und/oder Hydroxygruppen im Molekül des Ausgangsstoffes vorhanden sein. Falls die vorhandenen Schutzgruppen voneinander verschieden sind, können sie in vielen Fällen selektiv abgespalten werden (vgl. dazu: T.W. Greene, P.G.M. Wuts, Protective Groups in Organic Chemistry, 2. Aufl., Wiley, New York 1991 oder P.J. Kocienski, Protecting Groups, 1. Aufl., Georg Thieme Verlag, Stuttgart - New-York, 1994).

Der Ausdruck "Aminoschutzgruppe" ist allgemein bekannt und bezieht sich auf Gruppen, die geeignet sind, eine Aminogruppe vor chemischen Umsetzungen zu schützen (zu blockieren). Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, alicyclischen, aromatischen oder heterocyclischen Carbonsäuren oder Sulfonsäuren abgeleitete Acylgruppen sowie insbesondere Alkoxycarbonyl-, Alkenyloxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxy-carbonyl, BOC, 2-lodethoxycarbonyl; Alkenyloxycarbonyl wie Allyloxycarbonyl (Aloc), Aralkyloxycarbonyl wie CBZ (synonym mit Z), 4-Methoxybenzyloxycarbonyl (MOZ), 4-Nitro-benzyloxycarbonyl oder 9-fluorenylmethoxycarbonyl (Fmoc); 2-(Phenylsulfonyl)ethoxycarbonyl; Trimethylsilylethoxycarbonyl (Teoc) oder Arylsulfonyl wie 4-Methoxy-2,3,6-trimethylphenyl-sulfonyl (Mtr). Bevorzugte Aminoschutzgruppen sind BOC, Fmoc und Aloc, ferner CBZ, Benzyl und Acetyl. Besonders bevorzugte Schutzgruppen sind BOC und Fmoc.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen. Typisch für solche Gruppen sind die oben genannten unsubstituierten oder substituierten Aryl-, Aralkyl-, Aroyl- oder Acylgruppen, ferner auch Alkylgruppen, Alkyl-, Aryl- oder Aralkyl-silylgruppen oder O,O- oder O,S-Acetale. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Aralkylgruppen wie Benzyl, 4-Methoxybenzyl oder 2,4-Dimethoxybenzyl, Aroylgruppen wie Benzoyl oder p-Nitrobenzoyl, Acylgruppen wie Acetyl oder Pivaloyl, p-Toluolsulfonyl, Alkylgruppen wie Methyl oder tert.-Butyl, aber auch Allyl, Alkylsilylgruppen wie Trimethylsilyl (TMS), Triisopropylsilyl (TIPS), tert.-Butyldimethylsilyl (TBS) oder Triethylsilyl, Trimethylsilylethyl, Aralkylsilylgruppen wie tert.-Butyldiphenylsilyl (TBDPS), cyclische Acetale wie Isopropyliden-, Cyclopentyliden-, Cyclohexyliden-, Benzyliden-, p-Methoxybenzyliden- oder o,p-Dimethoxybenzylidenacetal, acyclische Acetale wie Tetrahydropyranyl (Thp), Methoxymethyl (MOM), Methoxyethoxymethyl (MEM), Benzyloxymethyl (BOM) oder Methylthiomethyl (MTM). Besonders bevorzugte Hydroxyschutzgruppen sind Benzyl, Acetyl, tert.-Butyl oder TBS.

Das In-Freiheit-Setzen der Verbindungen der Formel I aus ihren funktionellen Derivaten ist für die jeweils benutzte Schutzgruppe aus der Literatur bekannt (z.B. T.W. Greene, P.G.M. Wuts, Protective Groups in Organic Chemistry, 2. Aufl., Wiley, New York 1991 oder P.J. Kocienski, Protecting Groups, 1. Aufl., Georg Thieme Verlag, Stuttgart - New-York, 1994). Dabei kann man auch von an sich bekannten, hier nicht näher erwähnten Varianten Gebrauch machen.

Die Gruppen BOC und O-tert.-Butyl können z.B. bevorzugt mit TFA in Dichlormethan oder mit etwa 3 bis 5 N HCl in Dioxan bei 15-30°C abgespalten werden, die Fmoc-Gruppe mit einer etwa 5- bis 50%igen Lösung von Dimethylamin, Diethylamin oder Piperidin in DMF bei 15-30°C. Die Aloc-Gruppe läßt sich schonend unter Edelmetallkatalyse in Chloroform bei 20-30°C spalten. Ein bevorzugter Katalysator ist Tetrakis(triphenyl-phosphin)palladium(0).

Die Ausgangsverbindungen der Formel II bis VI und 1 bis 3 sind in der Regel bekannt. Sind sie neu, so können sie aber nach an sich bekannten Methoden hergestellt werden.

Die Verbindungen der Formel I können auch an fester Phase synthetisiert werden, wobei die Anbindung an die feste Phase über OH der Carboxylgruppe erfolgt. Bei Synthese an fester Phase ist die Carboxylgruppe substituiert mit OPoI, wobei Pol eine feste Phase ohne endständige funktionelle Gruppe bedeutet. Pol steht stellvertretend für das polymere Trägermaterial sowie für alle Atome der Ankergruppe einer festen Phase, bis auf die endständige funktionelle Gruppe. Die Ankergruppen einer festen Phase, auch Linker genannt, sind für die Anbindung der zu funktionalisierenden Verbindung an die feste Phase notwendig. Eine Zusammenfassung über Synthesen an fester Phase und den dazu einsetzbaren festen Phasen und/oder Linkern wird beispielsweise in Novabiochem - The Combinatorial Chemistry Catalog, March 99, Seiten S1-S72 gegeben.

Besonders geeignete feste Phasen für die Synthese der erfindungsgemäßen Verbindungen sind feste Phasen mit einer Hydroxygruppe als endständige Funktionalität, beispielsweise das Wang-Harz oder Polystyrene A OH.

Verbindungen der Formel II mit R¹ = Ar und R = OL, wobei L für Pol steht, werden beispielsweise nach folgendem Reaktionsschema 1 hergestellt, wobei SG₁ eine Aminoschutzgruppe bedeutet, wie zuvor beschrieben.

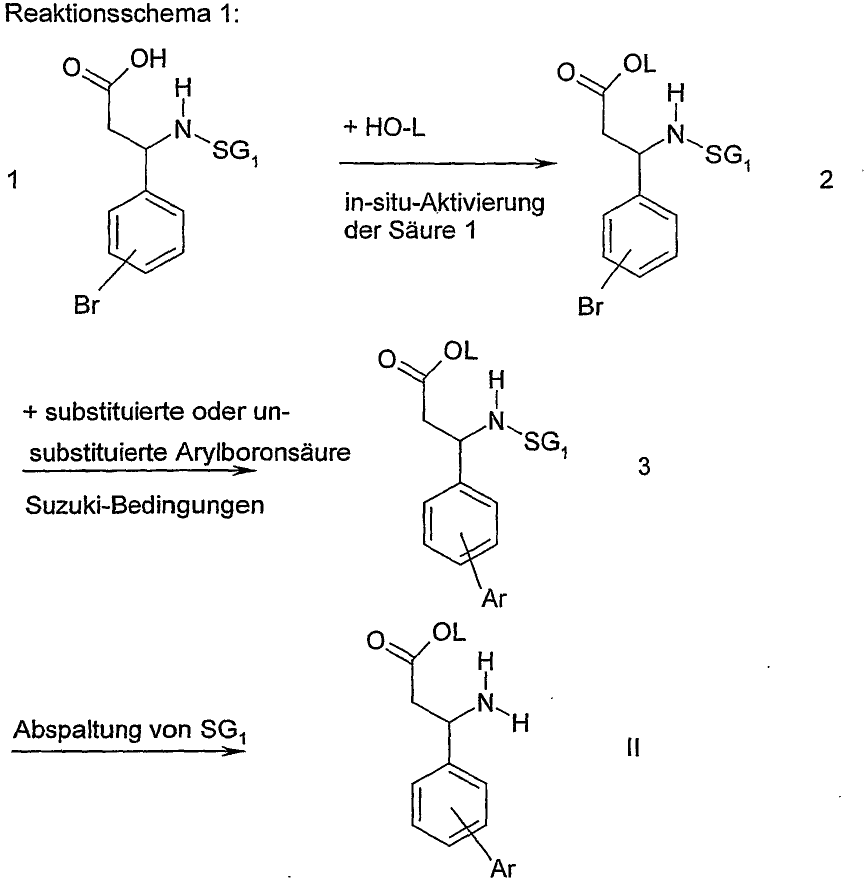

Die Bromphenyl-substituierte Carbonsäure 1 wird in situ nach bekannten Methoden aktiviert, beispielsweise durch Umsetzung mit Diisopropylcarbodiimid, und mit dem Alkohol HO-L umgesetzt, wobei L die oben angegebene Bedeutung besitzt. Die anschließende Kupplung der Verbindung 2 mit einer unsubstituierten oder substituierten Arylboronsäure unter Suzuki-Verbindungen erzeugt das Derivat 3. Die Abspaltung der Schutzgruppe SG₁ unter bekannten Bedingungen setzt eine Verbindung der Formel II frei.

Man führt die Suzuki-Reaktion zweckmäßig Palladium-vermittelt durch, bevorzugt durch Zugabe von Pd(PPh₃)₄, in Gegenwart einer Base wie Kaliumcarbonat in einem inerten Lösungsmittel oder Lösungsmittelgemisch z.B. DMF bei Temperaturen zwischen 0° und 150°, vorzugsweise zwischen 60° und 120°. Die Reaktionszeit liegt je nach den angewendeten Bedingungen zwischen einigen Minuten und mehreren Tagen. Die Boronsäurederivate können nach herkömmlichen Methoden hergestellt werden oder sind kommerziell erhältlich. Die Reaktionen können in Analogie zu den in Suzuki et al., J. Am. Chem. Soc. 1989, 111, 314ff. und in Suzuki et al. Chem. Rev. 1995, 95, 2457ff. angegebenen Methoden durchgeführt werden.

Verbindungen der Formel I werden durch eine peptidanaloge Kupplung der Verbindungen der Formel II mit einer Verbindung der Formel III oder durch peptidanaloge Kupplung der Verbindungen der Formel IV mit einer Verbindung der Formel V unter Standardbedingungen erhalten. Verbindungen der Formel III werden durch peptidanaloge Kupplung einer Verbindung der Formel V mit einer Aminoverbindung H₂N-C(R²,R^{2'})-COOSG² unter Standardbedingungen erhalten, wobei SG² eine Hydroxyschutzgruppe bedeutet wie zuvor beschrieben, die nach der Kupplung abgespaltet wird. Verbindungen der Formel IV werden durch peptidanaloge Kupplung einer Verbindung der Formel II mit einer Carboxyverbindung HOOC-C(R², R^{2'})-NHSG, unter Standardbedingungen erhalten, wobei SG₁ eine Aminoschutzgruppe bedeutet wie zuvor beschrieben, die nach der Kupplung abgespaltet wird.
Übliche Methoden der Peptidsynthese werden z.B. in Houben-Weyl, 1.c., Band 15/II, 1974, Seite 1 bis 806 beschrieben.

Die Kupplungsreaktion gelingt vorzugsweise in Gegenwart eines Dehydratisierungsmittels, z.B. eines Carbodiimids wie Dicyclohexylcarbodiimid (DCC), N-(3-Dimethylaminopropyl)-N'-ethyl-carbodiimid-hydrochlorid (EDC) oder Diisopropylcarbodiimid (DIC), ferner z.B. Propanphosphonsäureanhydrid (vgl. Angew. Chem. 1980, 92, 129), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, in Dimethylsulfoxid oder in Gegenwart dieser Lösungsmittel, bei Temperaturen zwischen etwa -10 und 40, vorzugsweise zwischen 0 und 30°. Die Reaktionszeit liegt je nach den angewendeten Bedingungen zwischen einigen Minuten und mehreren Tagen.
Als besonders vorteilhaft hat sich die Zugabe des Kupplungsreagenzes TBTU (O-(Benzotriazol-1-yl)-N,N,N',N'-tetramethyl-uronium-tetrafluoroborat) oder O-(Benzotriazol-1-yl)-N,N,N',N'-tetramethyl-uronium-hexafluorophosphat erwiesen, da in Gegenwart einer dieser Verbindungen nur eine geringe Racemisierung auftritt und keine cytotoxischen Nebenprodukte entstehen.

Anstelle von Verbindungen der Formeln III, V und/oder VI können auch Derivate von Verbindungen der Formel III, V und/oder VI, vorzugsweise eine voraktivierte Carbonsäure, oder ein Carbonsäurehalogenid, ein symmetrisches oder gemischtes Anhydrid oder ein Aktivester eingesetzt werden. Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart) beschrieben. Aktivierte Ester werden zweckmäßig in situ gebildet, z.B. durch Zusatz von HOBt (1-Hydroxybenzotriazol) oder N-Hydroxysuccinimid.

Die Umsetzung erfolgt in der Regel in einem inerten Lösungsmittel, bei Verwendung eines Carbonsäurehalogenids in Gegenwart eines säurebindenden Mittels vorzugsweise einer organischen Base wie Triethylamin, Dimethylanilin, Pyridin oder Chinolin.

Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Eine Base der Formel I kann mit einer Säure in das zugehörige Säureadditionssalz überführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, schweflige Säure, Dithionsäure, Salpetersäure, Halogenwasserstoffsäuren wie Chlorwasserstoffsäure oder Bromwasserstoffsäure, Phosphorsäuren wie z.B. Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Hexansäure, Octansäure, Decansäure, Hexadecansäure, Octadecansäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Benzolsulfonsäure, Trimethoxybenzoesäure, Adamantancarbonsäure, p-Toluolsulfonsäure, Glycolsäure, Embonsäure, Chlorphenoxyessigsäure, Asparaginsäure, Glutaminsäure, Prolin, Glyoxylsäure, Palmitinsäure, Parachlorphenoxyisobuttersäure, Cyclohexancarbonsäure, Glucose-1-phosphat, Naphthalin-mono- und disulfonsäuren oder Laurylschwefelsäure. Salze mit physiologisch nicht unbedenklichen Säuren, z.B. Pikrate, können zur Isolierung und/oder Aufreinigung der Verbindungen der Formel I verwendet werden. Andererseits können Verbindungen der Formel I mit Basen (z.B. Natrium-oder Kaliumhydroxid oder -carbonat) in die entsprechenden Metall-, insbesondere Alkalimetall- oder Erdalkalimetall- oder in die entsprechenden Ammoniumsalze umgewandelt werden.

Gegenstand der Erfindung sind auch die Verbindungen der Formel I, ihre Stereoisomere und ihre physiologisch unbedenklichen Salze oder Solvate als Arzneimittelwirkstoffe.

Weiterhin sind Gegenstand der Erfindung Verbindungen der Formel I, ihre Stereoisomere und ihre physiologisch unbedenklichen Salze oder Solvate als Integrininhibitoren.

Gegenstand der Erfindung sind auch die Verbindungen der Formel I, ihre Stereoisomere und ihre physiologisch unbedenklichen Salze oder Solvate zur Anwendung bei der Bekämpfung von Krankheiten.

Gegenstand der Erfindung sind ferner Arzneimittel, enthaltend mindestens eine Verbindung der Formel I, ihre Stereoisomere und/oder eines ihrer physiologisch unbedenklichen Salze oder Solvate. Hierzu können die Verbindungen der Formel I zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung ist ebenso die Verwendung von Verbindungen der Formel I, ihrer Stereoisomere und/oder ihrer physiologisch unbedenklichen Salze oder Solvate zur Herstellung eines Arzneimittels.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlenhydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks-und/oder mehrere weitere Wirkstoffe enthalten, z.B. ein oder mehrere Vitamine.

Für die Applikation als Inhalationsspray können Sprays verwendet werden, die den Wirkstoff entweder gelöst oder suspendiert in einem Treibgas oder Treibgasgemisch (z.B. CO₂ oder Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabreicht werden.

Die Verbindungen der Formel I, ihre Stereoisomere und/oder ihre physiologisch unbedenklichen Salze oder Solvate können als Arzneimittelwirkstoffe in der Human- und Veterinärmedizin eingesetzt werden, insbesondere zur Prophylaxe und/oder Therapie von Erkrankungen des Kreislaufs, Lungenfibrose, Lungenembolie, Thrombose, insbesondere tiefen Venenthrombosen, Herzinfarkt, Arteriosklerose, Aneurysma dissecans, vorübergehende ischämische Anfälle, Apoplexie, Angina pectoris, insbesondere instabile Angina pectoris, krankhaften Bindegewebsvermehrungen in Organen oder Fibrosen, insbesondere Lungenfibrose, aber auch Mucoviszidose, Hautfibrose, Leberfibrosen, Leberzirrhose, Blasenausgangsfibrosen, Nierenfibrose, Herzfibrose, infantile endocardiale Fibrose, Pankreasfibrose, Verhornungsstörungen der Haut, insbesondere Leukoplakien, Lichen planus und Plattenepithelkarzinome, Tumorerkrankungen, wie Tumorentwicklung Tumorangiogenese oder Tumormetastasierung, dabei soliden Tumoren und solchen des Blut oder Immunsystems, z.B. Tumore der Haut, Plattenepithelkarzinome, Tumore der Blutgefäße, des Magendarmtraktes, der Lunge, der Brust, der Leber, der Niere, der Milz, der Bauchspeicheldrüse, des Gehirns, der Hoden, des Ovars, der Gebärmutter, der Scheide, der Muskulatur, der Knochen, und solchen des Hals- und Kopf-Bereiches, osteolytischen Krankheiten wie Osteoporose, Hyperparathyreoidismus, Morbus Paget, maligne Hypercalcämie, inkompatibler Bluttransfusion, pathologisch angiogenen Krankheiten wie z.B. Entzündungen, ophthalmologischen Krankheiten, diabetischer Retinopathie, makularer Degeneration, Myopia, Corneatransplantation, okularer Histoplasmose, rheumatischer Arthritis, Osteoarthritis, rubeotischem Glaukom, ulcerativer Colitis, Morbus Crohn, Atherosklerose, Psoriasis, Restenose, insbesondere nach Angioplastie, Multiple Sklerose, Schwangerschaft, Absumptio placentaris, viraler Infektion, bakterieller Infektion, Pilzinfektion, Maul-und-Klauenseuche (FMDV), HIV, Anthrax, Candida albicans, bei parasitärem Befall, bei akutem Nierenversagen und bei der Wundheilung zur Unterstützung des Heilungsprozesses.

Bei viralem Befall erfolgt die Wirkung der erfindungsgemäßen Verbindungen insbesondere durch Inhibition oder Spaltung viraler Bindungen zwischen zell-vermittelten Integrin-bindenden Proteinen und der Virushülle oder indirekt durch Verhinderung der Aufnahme der Viren, die an extrazelluläre Matrixbestandteile gebunden sind, die als Integrine erkannt wurde, oder durch Spaltung von durch Integrine vermittelte Mechanismen, die mit der viralen Infektion einhergehen (J Virol 2000 Jun;74(11):4949-56, J Virol 2000 Aug;74(16):7298-306, J Virol 2001 May;75(9):4158-64, Virology. 2001 Sep 30;288(2):192-202. (FMDV), Virus Res. 2001 Jul;76(1):1-8 (Echovirus), J Biol Chem. 2001 Jul 13;276(28):26204-10. (HIV), Biochem Biophys Res Commun. 2001 May 11;283(3):668-73 (Papillomavirus), Proc Natl Acad Sci USA. 2000 Dec 19;97(26):14644-9 (Rotavirus)).

Bei bakteriellem Befall erfolgt die Wirkung insbesondere durch Inhibition der Bindung und/oder der Aufnahme der Bakterien oder bakteriellen Toxine oder der durch bakterielle Infektionen induzierten toxischen Produkten an Zellen über Integrin vermittelte Mechanismen (Nature 2001: Nov 8 : 225-229 (Anthrax), J Exp Med. 2001 May 7;193(9):1035-44 (Pertussis), Proc Natl Acad Sci U S A. 2000 Feb 29;97(5):2235-40 (grp A streptococcus), Infect Immun. 2000 Jan;68(1):72-9 (Pasturella Haemolytica Leukotoxin), J Biol Chem. 1997 Nov 28;272(48):30463-9. (RTX Leukotoxine)).

Bei parasitärem Befall erfolgt die Wirkung insbesondere durch Inhibition der Bindung und/oder Aufnahme der parasitären oder von Parasiten abgeleiteten oder induzierten Toxine an Zellen über an Integrine gerichtete Mechanismen (Infect Immun. 1999 Sep;67(9):4477-84.(Leishmania)).

Dabei werden die erfindungsgemäßen Substanzen in der Regel vorzugsweise in Dosierungen zwischen etwa 0,05 und 500 mg, insbesondere zwischen 0,5 und 100 mg pro Dosierungseinheit. Die tägliche Dosierung liegt vorzugsweise zwischen etwa 0,01 und 2 mg/kg Körpergewicht eingesetzt. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Ferner können die Verbindungen der Formel I als Integrinliganden zur Herstellung von Säulen für die Affinitätschromatographie zur Reindarstellung von Integrinen verwendet werden.
Der Ligand, d.h. eine Verbindung der Formel I, wird dabei über eine Ankerfunktion, z.B. die Carboxygruppe, an einen polymeren Träger kovalent gekuppelt.

Als polymere Trägermaterialien eignen sich die an sich in der Peptidchemie bekannten polymeren festen Phasen mit vorzugsweise hydrophilen Eigenschaften, beispielsweise quervernetzte Polyzucker wie Cellulose, Sepharose oder Sephadex^{R}, Acrylamide, Polymer auf Polyethylenglykol-oder Polystyrolbasis oder Tentakelpolymere^{R}.

Die Herstellung der Materialien für die Affinitätschromatographie zur Integrinreinigung erfolgt unter Bedingungen, wie sie für die Kondensation von Aminosäuren üblich und an sich bekannt sind.

Die Verbindungen der Formel I enthalten ein oder mehrere chirale Zentren und können daher in racemischer oder in optisch-aktiver Form vorliegen. Erhaltene Racemate können nach an sich bekannten Methoden mechanisch oder chemisch in die Enantiomeren getrennt werden. Vorzugsweise werden aus dem racemischen Gemisch durch Umsetzung mit einem optisch aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch aktiven Camphersulfonsäuren wie β-Camphersulfonsäure. Vorteilhaft ist auch eine Enantiomerentrennung mit Hilfe einer mit einem optisch aktiven Trennmittel (z.B. Dinitrobenzoyl-phenylglycin) gefüllten Säule; als Laufmittel eignet sich z.B. ein Gemisch Hexan/Isopropanol/Acetonitril, z.B. im Volumenverhältnis 82:15:3.

Natürlich ist es auch möglich, optisch aktive Verbindungen der Formel I nach den oben beschriebenen Methoden zu erhalten, indem man Ausgangsstoffe verwendet, die bereits optisch aktiv sind.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt duch Chromatographie an Kieselgel, durch präparative HPLC und/oder durch Kristallisation. Die gereinigten Verbindungen werden gegebenenfalls gefriergetrocknet.

Als Eluenten kommen Gradienten aus Acetonitril (B) mit 0,08 % TFA (Trifluoressigsäure) und Wasser (A) mit 0,1 % TFA zum Einsatz. Der Gradient wird in Volumenprozent Acetonitril angegeben.

Die HPLC-Analysen (Retentionszeit RT) erfolgten in den folgenden Systemen:
Säule 3 µm Silica-Rod mit einem 210-Sekunden Gradienten von 20 bis 100 % Wasser / Acetonitril / 0,01 % Trifluoressigsäure, bei 2,2 ml/min Fluss und Detektion bei 220 nm.
Oder
Säule Chromolith RP18e 100-4,6 mit einem 30 min Gradienten von 1 bis 75 % 0.014 M NaH2P04 / Isopropanol 1 ml/min Fluss und Detektion bei 220 nm.

Die durch präparative HPLC gereinigten Verbindungen werden als Trifluoracetate isoliert.
Massenspektrometrie (MS) mittels FAB (Fast Atom Bombardment): MS-FAB (M+H)⁺.

Die chromatographische Aufreinigungen wurden, sofern nicht anders bezeichnet, als offene Säulenchromatographie an Kieselgel (Korngroesse 0.064 - 0.2 mm) der Firma Merck KGaA durchgeführt. Als Laufmittel wurden Essigester und Heptan in reiner Form oder als Gemisch so verwendet, dass der R_{f}-Wert der zu isolierenden Verbindung 0.1 - 0.3 betrug.

Die Beispiele, ohne darauf beschränkt zu sein, erläutern die Erfindung.

Soweit die als Beispiele beschriebenen Verbindungen als verschiedene Stereoisomere vorliegen können und keine Angaben zur Stereochemie gegeben sind, liegen jeweils Gemische der Stereoisomere vor.

### Beispiel 1

### Synthese von 3-Biphenyl-4-yl-3-{3-(1,1-diphenyl-methylsulfanyl)-2-[2-(pyridin-2-ylamino)-ethoxycarbonylamino]-propanoylamino}-propionsäure

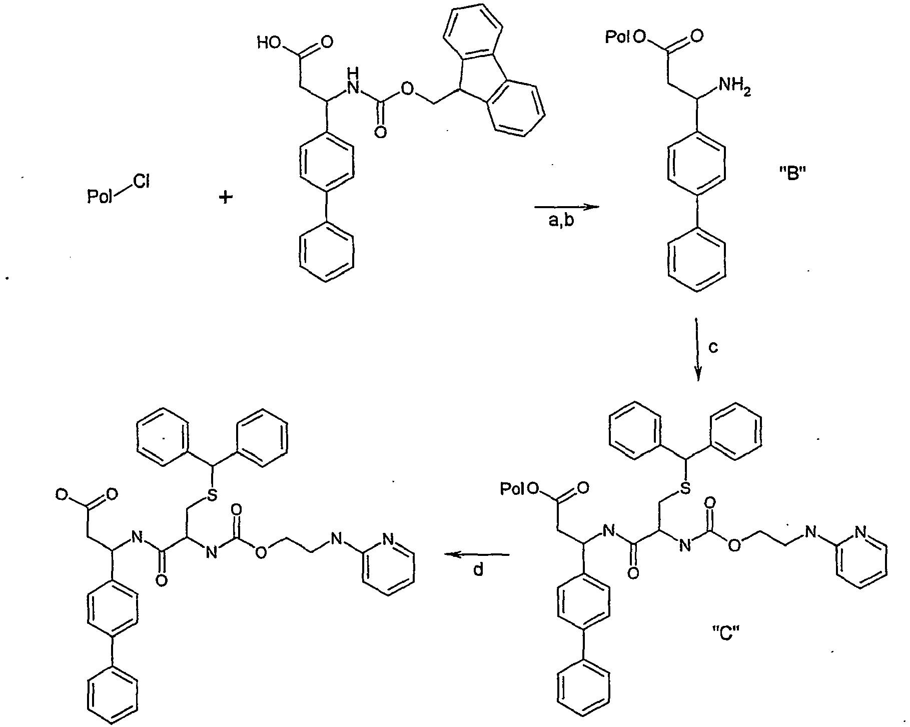

a 9,236 g 2-Chortritylchloridharz (Novabiochem) werden mit 80 ml Dichlormethan suspendiert und anschließend 3,9 ml Diisopropylethylamin zugegeben. Zu dieser Suspension wird eine Lösung von 7,00 g Fmocdiphenylaminopropionsäure in Dichormethan gegeben und anschließend 2 h bei RT geschüttelt. Zur Aufarbeitung wurde die feste Phase abfiltriert und je 3 mal mit Dichlormethan, DMF, Dichlormethan und Methanol gewaschen und in einem Vakuumtrockenschrank getrocknet.
b Die feste Phase wird mit DMF suspendiert und anschießend mit einer 50 %igen Lösung von Piperidin in DMF versetzt und 30 min bei RT geschüttelt. Anschließend wird die feste Phase abfiltriert und die gleiche Vorgehensweise zweimal wiederholt. Abschließend wird die feste Phase je dreimal mit DMF, Dichlormethan und Methanol gewaschen und in einem Vakuumtrockenschrank über Nacht getrocknet. Man erhält harzgebundene 3-Biphenyl-4-yl-3-aminopropionsäure "B".
c 300 mg feste Phase werden in 10 ml DMF suspendiert und mit 0,317 g 3-Benzhydrylsulfanyl-2-[2-(pyridin-2-ylamino)-ethoxycarbonylamino]-propionsäureethylester, 0,410 g Bromo-tris-pyrrolidino-phosphonium hexafluorophosphat sowie 0,307 ml Diisopropylethylamin versetzt und über Nacht bei Raumtemperatur stehen gelassen. Zur Aufarbeitung wird die feste Phase abfiltriert, zweimal mit DMF und je viermal mit DMF/Wasser (1/1, V/V), DMF, Dichlormethan und Methanol gewaschen und in einem Vakuumtrockenschrank getrocknet. Man erhält harzgebundene 3-Bipheny(-4-yl-3-{3-(1,1-diphenyl-methylsulfanyl)-2-[2-(pyridin-2-ylamino)-ethoxycarbonylamino]-propanoylamino}-propionsäure "C".
d 150 mg des Polymers werden in 1 ml Dichlormethan suspendiert und anschließend mit 3 ml einer 50 %igen Lösung von TFA in Dichlormethan versetzt und 1 h bei RT geschüttelt. Die feste Phase wurde durch Filtration entfernt und die Lösung im Vakuum zur Trockne eingeengt. Durch präparative HPLC erhält man 10 mg des gewünschten Produkts als Trifluoracetat (amorphe Festsubstanz).

Analog zu Beispiel 1 wird das Harz "B" mit 3-Benzhydrylsulfanyl-2-[2-(pyridin-2-ylamino)-propoxycarbonylamino]-propionsäureethylester umgesetzt. Man erhält 3-Biphenyl-4-yl-3-{3-(1,1-diphenyl-methylsulfanyl)-2-[3-(pyridin-2-ylamino)-propoxycarbonylamino]-propanoylamino}-propionsäure.
Durch präparative HPLC erhält man 3-Biphenyl-4-yl-3-{3-(1,1-diphenyl-methylsulfanyl)-2-[3-(pyridin-2-ylamino)-propoxycarbonylamino]-propanoylaminol-propionsäure Trifluoracetat, RT 1,925 min, FAB-MS (M+H)⁺ 689.

Analog zu Beispiel 1 wird das Harz "B" mit 3-Benzyloxy-2-[3-(pyridin-2-ylamino)-propoxycarbonylamino]-propionsäure umgesetzt. Man erhält 3-{3-Benzyloxy-2-[3-(pyridin-2-ylamino)-propoxycarbonylamino]-propanoylamino}-3-biphenyl-4-yl-propionsäure.
Durch präparative HPLC erhält man 3-{3-Benzyloxy-2-[3-(pyridin-2-ylamino)-propoxycarbonylamino]-propanoylamino}-3-biphenyl-4-yl-propionsäure Trifluoracetat, RT 1,641 min, FAB-MS (M+H)⁺ 597.

Analog zu Beispiel 1 wird das Harz "B" mit 3-Benzyloxy-2-[2-(pyridin-2-ylamino)-ethoxycarbonylamino]-propionsäure umgesetzt. Man erhält 3-{3-Benzyloxy-2-[2-(pyridin-2-ylamino)-ethoxycarbonylamino]-propanoylamino}-3-biphenyl-4-yl-propionsäure.
Durch präparative HPLC erhält man 3-{3-Benzyloxy-2-[2-(pyridin-2-ylamino)-ethoxycarbonylamino]-propanoylamino}-3-biphenyl-4-yl-propionsäure Trifluoracetat, RT 1,529 min (Gemisch), 1,556 (Diastereomerenpaar 1), 1,590 (Diastereomerenpaar 2), FAB-MS (M+H)⁺ 583 / 583 / 583.

Analog zu Beispiel 1 wird das Harz "B" mit 3-Benzyloxy-2-[2-(6-methylamino-pyridin-2-yl)-ethoxycarbonylamino]-propionsäure umgesetzt. Man erhält 3-{3-Benzyloxy-2-[2-(6-methylamino-pyridin-2-yl)-ethoxycarbonylamino]-propanoylamino}-3-biphenyl-4-yl-propionsäure. Durch präparative HPLC erhält man 3-{3-Benzyloxy-2-[2-(6-methylaminopyridin-2-yl)-ethoxycarbonylamino]-propanoylamino}-3-biphenyl-4-yl-propionsäure Trifluoracetat, RT 1,061 min, FAB-MS (M+H)⁺ 597.

Analog zu Beispiel 1 wird das Harz "B" mit 3-Benzyloxy-2-[3-(pyridin-2-ylamino)-propoxycarbonylamino]-propionsäure umgesetzt. Man erhält 3-{3-Benzyloxy-2-[3-(pyridin-2-ylamino)-propoxycarbonylamino]-propanoylamino}-3-biphenyl-4-yl-propionsäure.
Durch präparative HPLC erhält man 3-{3-Benzyloxy-2-[3-(pyridin-2-ylamino)-propoxycarbonylamino]-propanoylamino}-3-biphenyl-4-yl-propionsäure Trifluoracetat, RT 1,529 min (Gemisch), 1,674 (Diastereomerenpaar 1), 1,754 (Diastereomerenpaar 2), FAB-MS (M+H)⁺ 597 / 597.

Analog zu Beispiel 1 wird das Harz "B" mit 3-Benzyloxy-2-[2-(6-methylpyridin-2-ylamino)-ethoxycarbonylamino]-propionsäure umgesetzt. Man erhält 3-{3-Benzyloxy-2-[2-(6-methyl-pyridin-2-ylamino)-ethoxycarbonylamino]-propanoylamino}-3-biphenyl-4-yl-propionsäure. Durch präparative HPLC erhält man 3-{3-Benzyloxy-2-[2-(6-methyl-pyridin-2-ylamino)-ethoxycarbonylamino]-propanoylamino}-3-biphenyl-4-yl-propionsäure Trifluoracetat, RT 1,701 min, FAB-MS (M+H)⁺ 597.

### Beispiel 2

### Synthese von 3-{3-Benzyloxy-2-[3-(pyridin-2-ylamino)-propoxycarbonylamino]-propanoylamino}-3-(3,5-dichloro-phenyl)-propionsäureethylester

119,44 mg 3-Amino-3-(3,5-dichloro-phenyl)-propionsäureethylester, 0,164 g 3-Benzyioxy-2-[3-(pyridin-2-ylamino)-propoxycarbonylamino]-propionsäure, 10,00 ml DMF, 0,084 g N-(3-Dimethylaminopropyl)-N'-ethylcarbodiimid Hydrochlorid und 0,097 ml 4-Methylmorpholin werden zusammen unter Rühren zunächst 1,5 Stunden bei 50°C und anschließend über Nacht bei Raumtemperatur umgesetzt. Nach Abdestillation des DMF wird der Rückstand in Ethylacetat aufgenommen, mit Wasser gewaschen, bis zum Rückstand eingeengt und über eine kurze Kieselsäule chromatographisch aufgereinigt (Fließmittel Ethylacetat). Man erhält 3-{3-Benzyloxy-2-[3-(pyridin-2-ylamino)-propoxycarbonylamino]-propanoylamino}-3-(3,5-dichloro-phenyl)-propionsäureethylester, RT 1,552 min, FAB-MS (M+H)⁺ 618.

Analog zu Beispiel 2 werden 3-Amino-3-(3,5-dichloro-phenyl)-propionsäureethylester mit 3-Benzyloxy-2-[2-(6-methylamino-pyridin-2-ylamino)-ethoxycarbonylamino]-propionsäure umgesetzt und chromatographisch aufgereinigt. Man erhält 3-{3-Benzyloxy-2-[2-(6-methylamino-pyridin-2-yl)-ethoxycarbonylamino]-propanoylamino}-3-(3,5-dichloro-phenyl)-propionsäureethylester, RT 1,720 min, FAB-MS (M+H)⁺ 618.

Analog zu Beispiel 2 werden 3-Amino-3-(3,5-dichloro-phenyl)-propionsäureethylester mit 3-Benzyloxy-2-[2-(pyridin-2-ylamino)-ethoxycarbonylamino]-propionsäure umgesetzt und chromatographisch aufgereinigt. Man erhält 3-{3-Benzyloxy-2-[2-(pyridin-2-ylamino)-ethoxycarbonylamino]-propanoylamino}-3-(3,5-dichioro-phenyl)-propionsäureethylester, RT 1,712 min, FAB-MS (M+H)⁺ 604.

Analog zu Beispiel 2 werden 3-Amino-3-(3,5-dichloro-phenyl)-propionsäureethylester mit 3-Benzyloxy-2-[3-(pyridin-2-ylamino)-propoxycarbonylamino]-propionsäure umgesetzt und mittels präparativer HPLC aufgereinigt. Man erhält 3-(3-Benzyloxy-2-[3-(pyridin-2-ylamino)-propoxycarbonylamino]-propanoylamino)-3-(3,5-dichloro-phenyl)-propionsäure, RT 1,598 min, FAB-MS (M+H)⁺ 590.

Analog zu Beispiel 2 werden 3-Amino-3-(3,5-dichloro-phenyl)-propionsäureethylester mit 3-Benzyloxy-2-[2-(6-methyl-pyridin-2-ylamino)-ethoxycarbonylamino]-propionsäure umgesetzt und chromatographisch aufgereinigt. Man erhält 3-{3-Benzyloxy-2-[2-(6-methyl-pyridin-2-ylamino)-ethoxycarbonylamino]-propanoylamino}-3-(3,5-dichloro-phenyl)-propionsäureethylester, RT 1,765 min, FAB-MS (M+H)⁺ 618.

50,00 mg 3-{3-Benzyloxy-2-[2-(6-methyl-pyridin-2-ylamino)-ethoxycarbonylamino]-propanoylamino}-3-(3,5-dichloro-phenyl)-propionsäureethylester werden unter Rühren 2 Stunden 2 ml Ethanol und 0,50 ml 1 molare Natronlauge umgesetzt, mit 0.5 ml Eisessig angesäuert und bis zum Rückstand eingeengt. Man erhält 3-{3-Benzyloxy-2-[2-(6-methyl-pyridin-2-ylamino)-ethoxycarbonylamino]-propanoylamino}-3-(3,5-dichloro-phenyl)-propionsäure
Durch präparative HPLC erhält man 3-{3-Benzyloxy-2-[2-(6-methyl-pyridin-2-ylamino)-ethoxycarbonylamino]-propanoylamino}-3-(3,5-dichloro-phenyl)-propionsäure Trifluoracetat, RT 1,581 min, FAB-MS (M+H)⁺ 590.

Die nachfolgenden Beispiele betreffen pharmazeutische Zubereitungen:

### Beispiel A: Injektionsgläser

Eine Lösung von 100 g eines Wirkstoffes der Formel 1 und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H_{2O}, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 I isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I 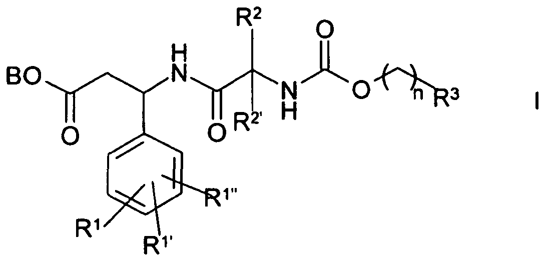 worin
R¹,R^{1'},R^{1"} H, A, Ar, Het¹, Hal, NO₂, CN, OR⁴, COA, NHCOA, NH(CHO), NR⁴, COOR⁴, CONHR⁴₂
R² A, Ar, (CH₂)ₘXA, (CH₂)ₘOH, (CH₂)mNH₂, (CH₂)ₘNHA, (CH₂)ₘNA₂, (CH₂)ₘNHCOA, (CH₂)ₘNO₂, (CH₂)ₘCOOR¹, (CH₂)ₘCONH₂, (CH₂)ₘX(CH₂)ₒAr, (CH₂)ₘX(CH₂)ₒCHAr₂, (CH₂)ₘX(CH₂)ₒCAr₃, (CH₂)ₘXCOYA, (CH₂)ₘXCOY(CH₂)ₒAr, (CH₂)ₘX(CH₂)ₒHet¹, (CH₂)ₘX(CH₂)ₒCHHet¹₂, (CH₂)ₘX(CH₂)_{O}CHet¹₃, (CH₂)ₘX(CH₂)ₒYA, (CH₂)ₘX(CH₂)ₒNHCOA, (CH₂)ₘNHCONHR^{2'}, (CH₂)ₘCH₂A, (CH₂)ₘCHA₂, (CH₂)ₘCA₃, (CH₂)ₘAr, (CH₂)ₘCHAr₂, (CH₂)ₘCAr₃, (CH₂)ₘHet¹, (CH₂)ₘCHHet¹₂, (CH₂)ₘCHet¹₃, (CH₂)ₘCycloalkyl, (CH₂)ₘ-NH-C(=NH)-NH₂, (CH₂)ₘ-(HN=)C-NH₂, wobei X und Y unabhängig voneinander S, O, S=O, SO₂ oder NH sein können, wobei wenn R² = (CH₂)ₘXCOYA oder (CH₂)ₘXCOY(CH₂)ₒAr ist, X und Y nicht S=O oder SO₂ sein können
R^{2'} H, A
R² und R^{2'} zusammen auch -(CH₂)ₚ- sein können
R³ A-C(=NH)-NH-, Het²- oder Het²-NH-, wobei die primären Aminogruppen auch mit konventionellen Aminoschutzgruppen versehen sein können,
R⁴ H, A, Het¹, Hal, NO₂, CN
A Alkyl mit 1 bis 8 C-Atomen
B H oder A
Ar unsubstituiertes oder ein- oder mehrfach durch Hal, A, OA, OH, CO-A, CN, COOA, COOH, CONH₂, CONHA, CONA₂, CF₃, OCF₃ oder NO₂ substituiertes Phenyl, Naphthyl, Anthranyl oder Biphenyl
Het¹ einen aromatischen ein- oder zweikernigen Heterocyclus mit 1 bis 3 N-, O- und / oder S-Atomen, der unsubstituiert oder ein- oder zweifach durch F, Cl, Br, A, OA, SA, OCF₃, -CO-A, CN, COOA, CONH₂, CONHA, CONA₂, NA₂ oder NO₂ substituiert sein kann
Het² einen ein- oder zweikernigen Heterocyclus mit 1 bis 4 N-Atomen, der unsubstituiert oder ein- oder zweifach durch NH₂, NHA oder A substituiert sein kann,
m 0, 1, 2, 3, 4, 5, 6, oder 8
n 1, 2, 3, 4, 5 oder 6
o 0, 1, 2 oder 3
p 2, 3, 4 oder 5
bedeutet,
deren Stereoisomere sowie deren physiologisch unbedenklichen Salze und Solvate

2. Verbindungen nach Anspruch 1, **dadurch gekennzeichnet, dass** diese
3-Biphenyl-4-yl-3-{3-(1,1-diphenyl-methylsulfanyl)-2-[2-(pyridin-2-ylamino)-ethoxycarbonylamino]-propanoylamino}-propionsäure
3-Biphenyl-4-yl-3-{3-(1,1-diphenyl-methylsulfanyl)-2-[3-(pyridin-2-ylamino)-propoxycarbonylamino]-propanoylamino}- propionsäure
3-{3-Benzyloxy-2-[3-(pyridin-2-ylamino)-propoxycarbonylamino]-propanoylamino}-3-biphenyl-4-yl-propionsäure
3-{3-Benzyloxy-2-[2-(pyridin-2-ylamino)-ethoxycarbonylamino]-propanoylamino}-3-biphenyl-4-yl-propionsäure
3-{3-Benzyloxy-2-[2-(6-methylamino-pyridin-2-yl)-ethoxycarbonylamino]-propanoylamino}-3-biphenyl-4-yl-propionsäure
3-{3-Benzyloxy-2-[3-(pyridin-2-ylamino)-propoxycarbonylamino]-propanoylamino}-3-biphenyl-4-yl-propionsäure
3-{3-Benzyloxy-2-[2-(6-methyl-pyridin-2-ylamino)-ethoxycarbonylamino]-propanoylamino}-3-biphenyl-4-yl-propionsäure
3-{3-Benzyloxy-2-[3-(pyridin-2-ylamino)-propoxycarbonylamino]-propanoylamino}-3-(3,5-dichloro-phenyl)-propionsäureethylester
3-{3-Benzyloxy-2-[2-(6-methylamino-pyridin-2-yl)-ethoxycarbonylamino]-propanoylamino}-3-(3,5-dichloro-phenyl)-propionsäureethylester
3-{3-Benzyloxy-2-[2-(pyridin-2-ylamino)-ethoxycarbonylamino]-propanoylamino}-3-(3,5-dichloro-phenyl)-propionsäureethylester
3-{3-Benzyloxy-2-[3-(pyridin-2-ylamino)-propoxycarbonylamino]-propanoylamino}-3-(3,5-dichloro-phenyl)-propionsäure (EMD 397966)
3-{3-Benzyloxy-2-[2-(6-methyl-pyridin-2-ylamino)-ethoxycarbonylamino]-propanoylamino}-3-(3,5-dichloro-phenyl)-propionsäureethylester
3-{3-Benzyloxy-2-[2-(6-methyl-pyridin-2-ylamino)-ethoxycarbonylamino]-propanoylamino}-3-(3,5-dichloro-phenyl)- propionsäure
sind,
deren Stereoisomere sowie deren physiologisch unbedenklichen Salze und Solvate.

3. Verfahren zur Herstellung der Verbindungen der Formel I nach Anspruch 1, deren Stereoisomere sowie ihrer Salze und Solvate, **dadurch gekennzeichnet, daß** man
(a) eine Verbindung der Formel II 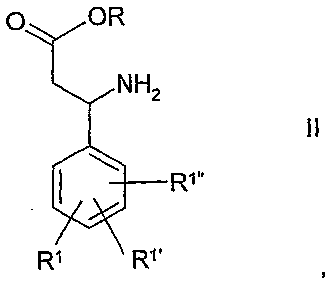 worin R eine Schutzgruppe ist und R¹, R^{1'}, R^{1"} die in Formel I angegebenen Bedeutungen haben und worin für den Fall dass R¹, R^{1'} und/oder R^{1"} freie Hydroxyl- oder Aminogruppen aufweisen diese durch jeweils durch eine Schutzgruppe geschützt vorliegt
mit einer Verbindung der Formel III 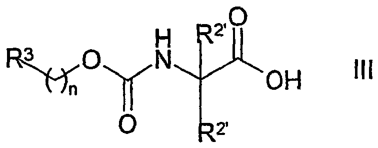 worin R², R^{2'}, R³ und n die in Formel I angegebenen Bedeutungen haben und worin für den Fall, dass R², R^{2'} und/oder R³ freie Hydroxyl- oder Aminogruppen enthalten, diese jeweils durch Schutzgruppen geschützt vorliegen
umsetzt
und die Schutzgruppe R sowie gegebenenfalls die an R¹, R^{1'}, R^{1"} R², R^{2'} und/oder R³ enthaltenen Schutzgruppen abspaltet,
oder
(b) eine Verbindung der Formel IV 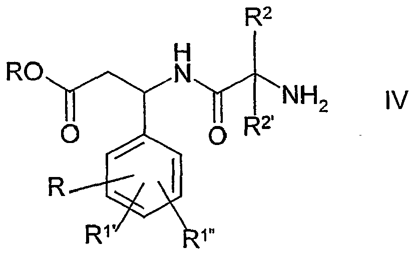 worin R eine Schutzgruppe ist und R¹, R^{1'}, R^{1"}, R² und R^{2'} die in Formel I angegebenen Bedeutungen haben und worin für den Fall, dass R¹, R^{1'}, R^{1"}, R² und/oder R^{2'} freie Hydroxyl- und/oder Aminogruppen enthalten, diese jeweils durch Schutzgruppen geschützt vorliegen
mit einer Verbindung der Formel V 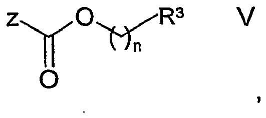 worin Z eine Abgangsgruppe wie z.B. Halogen oder eine reaktionsfähige veresterte OH-Gruppe bedeutet und n und R³ die in Formel I angegebenen Bedeutungen haben und worin für den Fall, dass R¹, R^{1'}, und/oder R^{1"} freie Hydroxyl- und/oder Aminogruppen enthalten, diese jeweils durch Schutzgruppen geschützt vorliegen, umsetzt
und die Schutzgruppe R sowie gegebenenfalls die an R¹, R^{1'}, R^{1"}, R², R^{2'} und/oder R³ enthaltenen Schutzgruppen abspaltet,
oder
(c) in einer Verbindung der Formel I einen oder mehrere Reste R¹, R^{1'}, R^{1"}, R², R^{2'} und/oder R³ in einen oder mehrere Reste R¹, R^{1'}, R^{1"}, R², R^{2'} und/oder R³ umwandelt,
indem man beispielsweise
i) eine Hydroxygruppe alkyliert,
ii) eine Estergruppe zu einer Carboxygruppe hydrolysiert,
iii) eine Carboxygruppe verestert,
iv) eine Aminogruppe alkyliert,
v) ein Arylbromid oder -iodid durch eine Suzuki-Kupplung mit Boronsäuren zu den entsprechenden Kupplungsprodukten umsetzt, oder
vi) eine Aminogruppe acyliert,
oder
(d) eine Verbindung der Formel II mit einer Verbindung der Formel VI 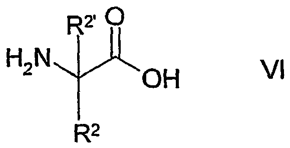 worin R² und R^{2'}die in Formel I angegebenen Bedeutungen haben und worin für den Fall, dass R² und/oder R^{2'} freie Hydroxyl- und/oder Aminogruppen enthalten, diese durch Schutzgruppen geschützt vorliegen
zu einer Verbindung der Formel IV umsetzt,
die erhaltene Verbindung der Formel IV gemäß (b) mit einer Verbindung der Formel V zu einer Verbindung der Formel I umsetzt und anschließend die Schutzgruppe R sowie gegebenenfalls die an R¹, R^{1'}, R^{1"}, R², R^{2'} und/oder R³ enthaltenen Schutzgruppen abspaltet,
und/oder
eine basische oder saure Verbindung der Formel I durch Behandeln mit einer Säure oder Base in eines ihrer Salze oder Solvate umwandelt.

4. Verbindungen der Formel I nach Anspruch 1 oder 2, ihre Stereoisomere und ihre physiologisch unbedenklichen Salze oder Solvate als Arzneimittelwirkstoffe.

5. Verbindungen der Formel I nach Anspruch 1 oder 2, ihre Stereoisomere und ihre physiologisch unbedenklichen Salze oder Solvate als Integrininhibitoren

6. Verbindungen der Formel I nach Anspruch 1 oder 2, ihre Stereoisomere und ihre physiologisch unbedenklichen Salze oder Solvate zur Anwendung bei der Bekämpfung von Krankheiten.

7. Arzneimittel, **dadurch gekennzeichnet, dass** dieses mindestens eine Verbindung der Formel I nach Anspruch 1 oder 2, deren Stereoisomere und/oder eines ihrer physiologisch unbedenklichen Salze oder Solvate enthält

8. Verwendung von Verbindungen der Formel I nach Anspruch 1 oder 2, ihrer Stereoisomere und/oder ihrer physiologisch unbedenklichen Salze oder Solvate zur Herstellung eines Arzneimittels.

9. Verwendung von Verbindungen der Formel I nach Anspruch 1 oder 2, ihrer Stereoisomere und/oder ihrer physiologisch unbedenklichen Salze oder Solvate zur Herstellung eines Arzneimittels zur Prophylaxe und/oder Therapie von Erkrankungen des Kreislaufs, Lungenfibrose, Lungenembolie, Thrombose, insbesondere tiefen Venenthrombosen, Herzinfarkt, Arteriosklerose, Aneurysma dissecans, vorübergehende ischämische Anfälle, Apoplexie, Angina pectoris, insbesondere instabile Angina pectoris, krankhaften Bindegewebsvermehrungen in Organen oder Fibrosen, insbesondere Lungenfibrose, aber auch Mucoviszidose, Hautfibrose, Leberfibrosen, Leberzirrhose, Blasenausgangsfibrosen, Nierenfibrose, Herzfibrose, infantile endocardiale Fibrose, Pankreasfibrose, Verhornungsstörungen der Haut, insbesondere Leukoplakien, Lichen planus und Plattenepithelkarzinome, Tumorerkrankungen, wie Tumorentwicklung Tumorangiogenese oder Tumormetastasierung, dabei soliden Tumoren und solchen des Blut oder Immunsystems, z.B. Tumore der Haut, Plattenepithelkarzinome, Tumore der Blutgefäße, des Magendarmtraktes, der Lunge, der Brust, der Leber, der Niere, der Milz, der Bauchspeicheldrüse, des Gehirns, der Hoden, des Ovars, der Gebärmutter, der Scheide, der Muskulatur, der Knochen, und solchen des Hals- und Kopf-Bereiches, osteolytischen Krankheiten wie Osteoporose, Hyperparathyreoidismus, Morbus Paget, maligne Hypercalcämie, inkompatibler Bluttransfusion, pathologisch angiogenen Krankheiten wie z.B. Entzündungen, ophthalmologischen Krankheiten, diabetischer Retinopathie, makularer Degeneration, Myopia, Corneatransplantation, okularer Histoplasmose, rheumatischer Arthritis, Osteoarthritis, rubeotischem Glaukom, ulcerativer Colitis, Morbus Crohn, Atherosklerose, Psoriasis, Restenose, insbesondere nach Angioplastie, Multiple Sklerose, Schwangerschaft, Absumptio placentaris, viraler Infektion, bakterieller Infektion, Pilzinfektion, Maul-und-Klauenseuche (FMDV), HIV, Anthrax, Candida albicans, bei parasitärem Befall, bei akutem Nierenversagen und bei der Wundheilung zur Unterstützung des Heilungsprozesses.

## Claims

1. Compounds of the formula I 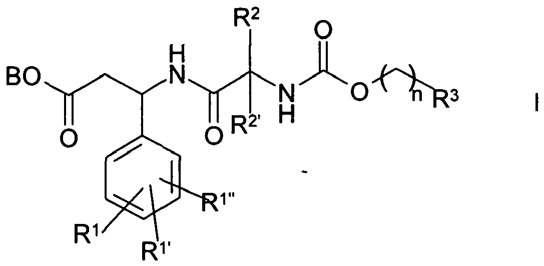 in which
R¹, R^{1'}, R^{1"} denote H, A, Ar, Het¹, Hal, NO₂, CN, OR⁴, COA, NHCOA, NH(CHO), NR⁴, COOR⁴, CONHR⁴₂
R² denotes A, Ar, (CH₂)ₘXA, (CH₂)ₘOH, (CH₂)ₘNH₂, (CH₂)ₘNHA, (CH₂)ₘNA₂, (CH₂)ₘNHCOA, (CH₂)ₘNO₂, (CH₂)ₘCOOR¹, (CH₂)ₘCONH₂, (CH₂)ₘX(CH₂)ₒAr, (CH₂)ₘX(CH₂)ₒCHAr₂, (CH₂)ₘX(CH₂)ₒCAr₃, (CH₂)ₘXCOYA, (CH₂)ₘXCOY(CH₂)ₒAr, (CH₂)ₘX(CH₂)ₒHet¹, (CH₂)ₘX(CH₂)ₒCHHet¹₂, (CH₂)ₘX(CH₂)ₒCHet¹₃, (CH₂)ₘX(CH2)ₒYA, (CH₂)ₘX(CH₂)oNHCOA, (CH₂)ₘNHCONHR^{2'}, (CH₂)ₘCH₂A, (CH₂)ₘCHA₂, (CH₂)ₘCA₃, (CH₂)ₘAr, (CH₂)ₘCHAr₂, (CH₂)ₘCAr₃, (CH₂)ₘHet¹, (CH₂)ₘCHHet¹₂, (CH₂)ₘCHet¹₃, (CH₂)ₘcycloalkyl, (CH₂)ₘ-NH-C(=NH)-NH₂,, (CH₂)ₘ-(HN=)C-NH₂,
R^{2'} where X and Y, independently of one another, can be S, O, S=O, SO₂ or NH, where, if R² = (CH₂)ₘXCOYA or (CH₂)ₘXCOY(CH₂)ₒAr, X and Y cannot be S=O or SO₂ denotes H, A
R² and R^{2'} together may alternatively be -(CH₂)ₚ-
R³ denotes A-C(=NH)-NH-, Het²- or Het²-NH-, where the primary amino groups may also be provided with conventional amino-protecting groups,
R⁴ denotes H, A, Het¹, Hal, NO₂, CN
A denotes alkyl having 1 to 8 C atoms
B denotes H or A
Ar denotes phenyl, naphthyl, anthranyl or biphenyl, each of which is unsubstituted or mono- or polysubstituted by Hal, A, OA, OH, CO-A, CN, COOA, COOH, CONH₂, CONHA, CONA₂, CF₃, OCF₃ or NO₂
Het¹ denotes an aromatic mono- or bicyclic heterocycle having 1 to 3 N, O and/or S atoms, which may be unsubstituted or mono- or disubstituted by F, Cl, Br, A, OA, SA, OCF₃, -CO-A, CN, COOA, CONH₂, CONHA, CONA₂, NA₂ or NO₂
Het² denotes a mono- or bicyclic heterocyclic radical having 1 to 4 N atoms, which may be unsubstituted or mono- or disubstituted by NH₂, NHA or A,
m denotes 0, 1, 2, 3, 4, 5, 6 or 8
n denotes 1, 2, 3, 4, 5 or 6
o denotes 0, 1, 2 or 3
p denotes 2, 3, 4 or 5,
stereoisomers thereof and physiologically acceptable salts and solvates thereof.

2. Compounds according to Claim 1, **characterised in that** these are
3-biphenyl-4-yl-3-{3-(1,1-diphenylmethylsulfanyl)-2-[2-(pyridin-2-ylamino)ethoxycarbonylamino]propanoylamino}propionic acid
3-biphenyl-4-yl-3-{3-(1,1-diphenylmethylsulfanyl)-2-[3-(pyridin-2-ylamino)propoxycarbonylamino]propanoylamino}propionic acid
3-{3-benzyloxy-2-[3-(pyridin-2-ylamino)propoxycarbonylamino]propanoylamino}-3-biphenyl-4-ylpropionic acid
3-{3-benzyloxy-2-[2-(pyridin-2-ylamino)ethoxycarbonylamino]propanoylamino}-3-biphenyl-4-ylpropionic acid
3-{3-benzyloxy-2-[2-(6-methylaminopyridin-2-yl)ethoxycarbonylamino]-propanoylamino}-3-biphenyl-4-ylpropionic acid
3-{3-benzyloxy-2-[3-(pyridin-2-ylamino)propoxycarbonylamino]propanoylamino}-3-biphenyl-4-ylpropionic acid
3-{3-benzyloxy-2-[2-(6-methylpyridin-2-ylamino)ethoxycarbonylamino]-propanoylamino}-3-biphenyl-4-ylpropionic acid
ethyl 3-{3-benzyloxy-2-[3-(pyridin-2-ylamino)propoxycarbonylamino]-propanoylamino}-3-(3,5-dichlorophenyl)propionate
ethyl 3-{3-benzyloxy-2-[2-(6-methylaminopyridin-2-yl)ethoxycarbonylamino]propanoylamino}-3-(3,5-dichlorophenyl)propionate
ethyl 3-{3-benzyloxy-2-[2-(pyridin-2-ylamino)ethoxycarbonylamino]-propanoylamino}-3-(3,5-dichlorophenyl)propionate
3-{3-benzyloxy-2-[3-(pyridin-2-ylamino)propoxycarbonylamino]propanoylamino}-3-(3,5-dichlorophenyl)propionic acid (EMD 397966)
ethyl 3-{3-benzyloxy-2-[2-(6-methylpyridin-2-ylamino)ethoxycarbonylamino]propanoylamino}-3-(3,5-dichlorophenyl)propionate
3-{3-benzyloxy-2-[2-(6-methylpyridin-2-ylamino)ethoxycarbonylamino]-propanoylamino}-3-(3,5-dichlorophenyl)propionic acid,
stereoisomers thereof and physiologically acceptable salts and solvates thereof.

3. Process for the preparation of the compounds of the formula I according to Claim 1, stereoisomers thereof and salts and solvates thereof, **characterised in that**
(a) a compound of the formula II 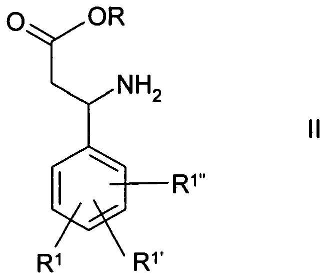 in which R is a protecting group and R¹, R^{1'}, R^{1"} have the meanings indicated in formula I and in which, in the case where R¹, R^{1'} and/or R^{1"} contain free hydroxyl or amino groups, these are in each case protected by a protecting group
is reacted with a compound of the formula III 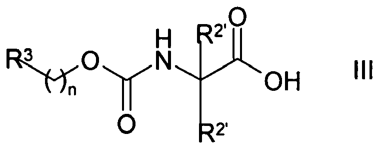 in which R², R^{2'}, R³ and n have the meanings indicated in formula I and in which, in the case where R², R^{2'} and/or R³ contain free hydroxyl or amino groups, these are in each case protected by protecting groups
and the protecting group R and any protecting groups present on R¹, R^{1'}, R^{1"}, R², R^{2'} and/or R³ are cleaved off,
or
(b) a compound of the formula IV 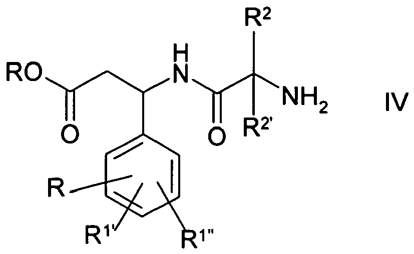 in which R is a protecting group and R¹, R^{1'}, R^{1"}, R² and R^{2'} have the meanings indicated in formula I and in which, in the case where R¹, R^{1'}, R^{1"}, R² and/or R^{2'} contain free hydroxyl and/or amino groups, these are in each case protected by protecting groups
is reacted with a compound of the formula V 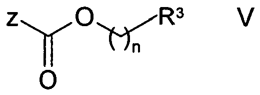 in which Z denotes a leaving group, such as, for example, halogen, or a reactive esterified OH group, and n and R³ have the meanings indicated in formula I and in which, in the case where R¹, R^{1'} and/or R^{1"} contain free hydroxyl and/or amino groups, these are in each case protected by protecting groups
and the protecting group R and any protecting groups present on R¹, R^{1'}, R^{1"}, R², R^{2'} and/or R³ are cleaved off,
or
(c) one or more radicals R¹, R^{1'}, R^{1"}, R², R^{2'} and/or R³ in a compound of the formula I are converted into one or more radicals R¹, R^{1'}, R^{1"}, R², R^{2'} and/or R³
by, for example,
i) alkylating a hydroxyl group,
ii) hydrolysing an ester group to a carboxyl group,
iii) esterifying a carboxyl group,
iv) alkylating an amino group,
v) reacting an aryl bromide or iodide with boronic acids by a Suzuki coupling to give the corresponding coupling products, or
vi) acylating an amino group,
or
(d) a compound of the formula II is reacted with a compound of the formula VI 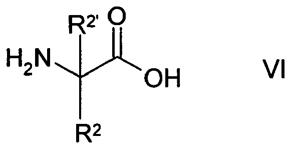 in which R² and R^{2'} have the meanings indicated in formula I and in which, in the case where R² and/or R^{2'} contain free hydroxyl and/or amino groups, these are protected by protecting groups to give a compound of the formula IV,
the resultant compound of the formula IV is reacted with a compound of the formula V as described in (b) to give a compound of the formula I
and the protecting group R and any protecting groups present on R¹, R^{1'}, R^{1"}, R², R^{2'} and/or R³ are subsequently cleaved off,
and/or
a basic or acidic compound of the formula I is converted into one of its salts or solvates by treatment with an acid or base.

4. Compounds of the formula I according to Claim 1 or 2, stereoisomers thereof and physiologically acceptable salts or solvates thereof as medicament active compounds.

5. Compounds of the formula I according to Claim 1 or 2, stereoisomers thereof and physiologically acceptable salts or solvates thereof as integrin inhibitors.

6. Compounds of the formula I according to Claim 1 or 2, stereoisomers thereof and physiologically acceptable salts or solvates thereof for use in combating diseases.

7. Medicament, **characterised in that** it comprises at least one compound of the formula I according to Claim 1 or 2, stereoisomers thereof and/or one of its physiologically acceptable salts or solvates.

8. Use of compounds of the formula I according to Claim 1 or 2, stereoisomers thereof and/or physiologically acceptable salts or solvates thereof for the preparation of a medicament.

9. Use of compounds of the formula I according to Claim 1 or 2, stereoisomers thereof and/or physiologically acceptable salts or solvates thereof for the preparation of a medicament for the prophylaxis and/or therapy of circulation disorders, pulmonary fibrosis, pulmonary embolism, thrombosis, in particular deep-vein thromboses, cardiac infarction, arteriosclerosis, dissecting aneurysm, transient ischaemic attacks, apoplexy, angina pectoris, in particular unstable angina pectoris, pathological connective tissue proliferation in organs or fibroses, in particular pulmonary fibrosis, but also cystic fibrosis, dermatofibrosis, hepatic fibroses, liver cirrhosis, urethrofibroses, renal fibrosis, cardiac fibrosis, infantile endocardial fibrosis, pancreatic fibrosis, hornification disorder of the skin, in particular leukoplakias, lichen planus and squamous cell carcinomas, tumour diseases, such as tumour development, tumour angiogenesis or tumour metastasis, of solid tumours and those of the blood or immune system, for example tumours of the skin, squamous cell carcinomas, tumours of the blood vessels, of the gastrointestinal tract, of the lung, of the breast, of the liver, of the kidney, of the spleen, of the pancreas, of the brain, of the testicles, of the ovary, of the womb, of the vagina, of the muscles, of the bones, and those of the neck and head area, osteolytic diseases, such as osteoporosis, hyperparathyroidism, Paget's disease, malignant hypercalcaemia, incompatible blood transfusion, pathologically angiogenic diseases, such as, for example, inflammation, ophthalmological diseases, diabetic retinopathy, macular degeneration, myopia, corneal transplant, ocular histoplasmosis, rheumatic arthritis, osteoarthritis, rubeotic glaucoma, ulcerative colitis, Crohn's disease, atherosclerosis, psoriasis, restenosis, in particular after angioplasty, multiple sclerosis, pregnancy, absumptio placentaris, viral infection, bacterial infection, fungal infection, foot-and-mouth disease (FMDV), HIV, anthrax, candida albicans, in the case of parasitic infestation, in the case of acute kidney failure and in the case of wound healing for supporting the healing process.

## Revendications

1. Composés de la formule I 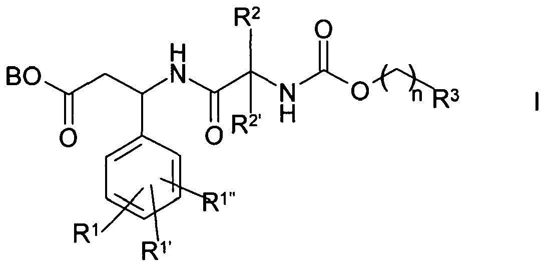 dans laquelle
R¹, R^{1'}, R^{1"} représentent H, A, Ar, Het¹, Hal, NO₂, CN, OR⁴, COA, NHCOA, NH(CHO), NR⁴, COOR⁴, CONHR⁴₂
R² représente A, Ar, (CH₂)ₘXA, (CH₂)ₘOH, (CH₂)ₘNH₂, (CH₂)ₘNHA, (CH₂)ₘNA₂, (CH₂)ₘNHCOA, (CH₂)ₘNO₂, (CH₂)ₘCOOR¹, (CH₂)ₘCONH₂, (CH₂)ₘX(CH₂)ₒAr, (CH₂)ₘX(CH₂)ₒCHAr₂, (CH₂)ₘX(CH₂)ₒCAr₃, (CH₂)ₘXCOYA, (CH₂)ₘXCOY(CH₂)ₒAr, (CH₂)ₘX(CH₂)ₒHet¹, (CH₂)ₘX(CH₂)ₒCHHet¹₂, (CH₂)ₘX(CH₂)ₒCHet¹₃, (CH₂)ₘX(CH₂)ₒYA, (CH₂)ₘX(CH₂)ₒNHCOA, (CH₂)ₘNHCONHR^{2'}, (CH₂)ₘCH₂A, (CH₂)ₘCHA₂, (CH₂)ₘCA₃, (CH₂)ₘAr, (CH₂)ₘCHAr₂, (CH₂)ₘCAr₃, (CH₂)mHet¹, (CH₂)ₘCHHet¹₂, (CH₂)ₘCHet¹₃, (CH₂)ₘcycloalkyle, (CH₂)ₘ-NH-C(=NH)-NH₂, (CH₂)m-(HN=)C-NH₂, où X et Y, indépendamment l'un de l'autre, peuvent être S, O, S=O, SO₂ ou NH, où, si R² = (CH₂)ₘXCOYA ou (CH₂)ₘXCOY(CH₂)ₒAr, X et Y ne peuvent pas être S=O ou SO₂
R^{2'} représente H, A
R² et R^{2'} peuvent être alternativement ensemble -(CH₂)ₚ-
R³ représente A-C(=NH)-NH-, Het²- ou Het²-NH-, où les groupes amino primaires peuvent également être munis de groupes de protection d'amino classiques,
R⁴ représente H, A, Het¹, Hal, NO₂, CN
A représente alkyle comportant de 1 à 8 atomes de C
B représente H ou A
Ar représente phényle, naphtyle, anthranyle ou biphényle, dont chacun est non substitué ou mono- ou polysubstitué par Hal, A, OA, OH, CO-A, CN, COOA, COOH, CONH₂, CONHA, CONA₂, CF₃, OCF₃ ou NO₂
Het¹ représente un hétérocycle mono- ou bicyclique aromatique comportant de 1 à 3 atomes de N, de O et/ou de S, lequel peut être non substitué ou mono- ou disubstitué par F, Cl, Br, A, OA, SA, OCF₃, -CO-A, CN, COOA, CONH₂, CONHA, CONA₂, NA₂ ou NO₂
Het² représente un radical hétérocyclique mono- ou bicyclique comportant de 1 à 4 atomes de N, lequel peut être non substitué ou mono- ou disubstitué par NH₂, NHA ou A,
m représente 0, 1, 2, 3, 4, 5, 6 ou 8
n représente 1, 2, 3, 4, 5 ou 6
o représente 0, 1, 2 ou 3
p représente 2, 3, 4 ou 5,
leurs stéréo-isomères et leurs sels et solvats physiologiquement acceptables.

2. Composés selon la revendication 1, **caractérisés en ce qu'**il s'agit de
l'acide 3-biphényl-4-yl-3-{3-(1,1-diphénylméthylsulfanyl)-2-[2-(pyridin-2-ylamino)éthoxycarbonylamino]propanoylamino}propionique
l'acide 3-biphényl-4-yl-3-{3-(1,1-diphénylméthylsulfanyl)-2-[3-(pyridin-2-ylamino)propoxycarbonylamino]propanoylamino}propionique
l'acide 3-{3-benzyloxy-2-[3-(pyridin-2-ylamino)propoxycarbonylamino]-propanoylamino}-3-biphényl-4-ylpropionique
l'acide 3-{3-benzyloxy-2-[2-(pyridin-2-ylamino)éthoxycarbonylamino]-propanoylamino}-3-biphényl-4-ylpropionique
l'acide 3-{3-benzyloxy-2-[2-(6-méthylaminopyridin-2-yl)éthoxycarbonylamino]propanoylamino}-3-biphényl-4-ylpropionique
l'acide 3-{3-benzyloxy-2-[3-(pyridin-2-ylamino)propoxycarbonylamino]-propanoylamino}-3-biphényl-4-ylpropionique
l'acide 3-{3-benzyloxy-2-[2-(6-méthylpyridin-2-ylamino)éthoxycarbonylamino]propanoylamino}-3-biphényl-4-ylpropionique
le 3-{3-benzyloxy-2-[3-(pyridin-2-ylamino)propoxycarbonylamino]-propanoylamino}-3-(3,5-dichlorophényl)propionate d'éthyle
le 3-{3-benzyloxy-2-[2-(6-méthylaminopyridin-2-yl)éthoxycarbonylamino]propanoylamino}-3-(3,5-dichlorophényl)propionate d'éthyle
le 3-{3-benzyloxy-2-[2-(pyridin-2-ylamino)éthoxycarbonylamino]-propanoylamino}-3-(3,5-dichlorophényl)propionate d'éthyle
l'acide 3-{3-benzyloxy-2-[3-(pyridin-2-ylamino)propoxycarbonylamino]propanoylamino}-3-(3,5-dichlorophényl)propionique (EMD 397966)
le 3-{3-benzyloxy-2-[2-(6-méthylpyridin-2-ylamino)éthoxycarbonylamino]propanoylamino}-3-(3,5-dichlorophényl)propionate d'éthyle
l'acide 3-{3-benzyloxy-2-[2-(6-méthylpyridin-2-ylamino)éthoxycarbonylamino]propanoylamino}-3-(3,5-dichlorophényl)propionique
leurs stéréo-isomères et leurs sels et solvats physiologiquement acceptables.

3. Procédé pour la préparation des composés de la formule I selon la revendication 1, de leurs stéréo-isomères et de leurs sels et solvats, **caractérisé en ce que**
(a) un composé de la formule II 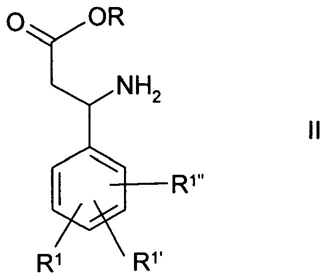 dans laquelle R est un groupe de protection et R¹, R^{1'}, R^{1"} présentent les significations indiquées selon la formule I et dans laquelle, dans le cas où R¹, R^{1'} et/ou R^{1"} contiennent des groupes hydroxyle ou amino libres, ceux-ci sont dans chaque cas protégés par des groupes de protection
est amené à réagir avec un composé de la formule III 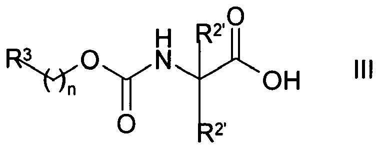 dans laquelle R², R^{2'}, R³ et n présentent les significations indiquées selon la formule I et dans laquelle, dans le cas où R², R^{2'} et/ou R³ contiennent des groupes hydroxyle ou amino libres, ceux-ci sont dans chaque cas protégés par des groupes de protection et le groupe de protection R et n'importe quels groupes de protection présents sur R¹, R^{1'} R^{1"}, R², R^{2'} et/ou R³ sont éliminés,
ou
(b) un composé de la formule IV 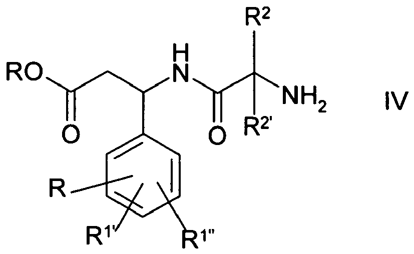 dans laquelle R est un groupe de protection et R¹, R^{1'}, R^{1"}, R² et R^{2'} présentent les significations indiquées selon la formule I et dans laquelle, dans le cas où R¹, R^{1'}, R^{1"}, R² et/ou R^{2'} contiennent des groupes hydroxyle et/ou amino libres, ceux-ci sont dans chaque cas protégés par des groupes de protection
est amené à réagir avec un composé de la formule V 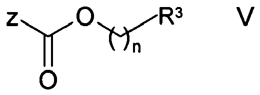 dans laquelle Z représente un groupe partant, tel que, par exemple, halogène, ou un groupe OH estérifié réactif, et n et R³ présentent les significations indiquées selon la formule I et dans laquelle, dans le cas où R¹, R^{1'} et/ou R^{1"} contiennent des groupes hydroxyle et/ou amino libres, ceux-ci sont dans chaque cas protégés par un groupe de protection
et le groupe de protection R et n'importe quels groupes de protection présents sur R¹, R^{1'}, R^{1"}, R², R^{2'} et/ou R³ sont éliminés,
ou
(c) un ou plusieurs radicaux R¹, R^{1'}, R^{1"}, R², R^{2'} et/ou R³ dans un composé de la formule I sont convertis selon un ou plusieurs radicaux R¹, R^{1'}, R^{1"}, R², R^{2'} et/ou R³
par, par exemple,
i) l'alkylation d'un groupe hydroxyle,
ii) l'hydrolyse d'un groupe ester selon un groupe carboxyle,
iii) l'estérification d'un groupe carboxyle,
iv) l'alkylation d'un groupe amino,
v) la réaction d'un bromure ou iodure d'aryle avec des acides boroniques par un couplage de Suzuki pour obtenir les produits de couplage correspondants, ou
vi) l'acylation d'un groupe amino,
ou
(d) un composé de la formule II est amené à réagir avec un composé de la formule VI 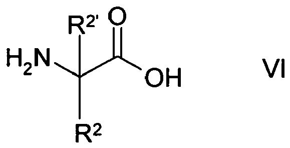 dans laquelle R² et R^{2'} présentent les significations indiquées selon la formule I et dans laquelle, dans le cas où R² et/ou R^{2'} contient des groupes hydroxyle et/ou amino libres, ceux-ci sont dans chaque cas protégés par des groupes de protection pour obtenir un composé de la formule IV,
le composé résultant de la formule IV est amené à réagir avec un composé de la formule V comme décrit selon (b) afin d'obtenir un composé de la formule I
et le groupe de protection R et n'importe quels groupes de protection présents sur R¹, R^{1'}, R^{1"}, R², R^{2'} et/ou R³ sont ensuite éliminés,
et/ou
un composé basique ou acide de la formule I est converti selon l'un de ses sels ou solvats par traitement avec un acide ou une base.

4. Composés de la formule I selon la revendication 1 ou 2, leurs stéréo-isomères et leurs sels ou solvats physiologiquement acceptables en tant que composés actifs de médicaments.

5. Composés de la formule I selon la revendication 1 ou 2, leurs stéréo-isomères et leurs sels ou solvats physiologiquement acceptables en tant qu'inhibiteurs d'intégrine.

6. Composés de formule I selon la revendication 1 ou 2, leurs stéréo-isomères et leurs sels ou solvats physiologiquement acceptables pour une utilisation dans la lutte contre des maladies.

7. Médicament, **caractérisé en ce qu'**il comprend au moins un composé de la formule I selon la revendication 1 ou 2, ses stéréo-isomères et/ou l'un de ses sels ou solvats physiologiquement acceptables.

8. Utilisation de composés de la formule I selon la revendication 1 ou 2, de leurs stéréo-isomères et/ou de leurs sels ou solvats physiologiquement acceptables pour la préparation d'un médicament.

9. Utilisation de composés de la formule I selon la revendication 1 ou 2, de leurs stéréo-isomères et/ou de leurs sels ou solvats physiologiquement acceptables pour la préparation d'un médicament pour la prophylaxie et/ou la thérapie de troubles circulatoires, de la fibrose pulmonaire, de l'embolie pulmonaire, de la thrombose, en particulier la thrombose veineuse profonde, de l'infarctus du myocarde, de l'artériosclérose, de l'anévrisme disséquant, des accidents ischémiques transitoires, de l'apoplexie, de l'angine de poitrine, en particulier l'angine de poitrine instable, de la prolifération du tissu conjonctif pathologique dans les organes ou de la fibrose, en particulier de la fibrose pulmonaire, mais également de la mucoviscidose, de la dermatofibrose, de la fibrose hépatique, de la cirrhose du foie, de l'urétrofibrose, de la fibrose rénale, de la fibrose cardiaque, de la fibrose endocardique infantile, de la fibrose pancréatique, de la perturbation de la kératinisation de la peau, en particulier de la leucoplasie, du lichen plan et du carcinome malpighien, des maladies tumorales, telles que du développement de tumeurs, de l'angiogénèse tumorale ou de la métastase tumorale, de tumeurs solides et de celles du système sanguin ou immunitaire, par exemple des tumeurs de la peau, du carcinome malpighien, des tumeurs des vaisseaux sanguins, du tractus gastro-intestinal, du poumon, du sein, du foie, du rein, de la rate, du pancréas, du cerveau, des testicules, de l'ovaire, de l'utérus, du vagin, des muscles, des os, et celles de la gorge et de la région de la tête, des maladies ostéolytiques, telles que de l'ostéoporose, de l'hyperparathyroïdisme, de la maladie de Paget, de l'hypercalcémie maline, de l'incompatibilité transfusionnelle sanguine, des troubles pathologiquement angiogènes, tels que par exemple des inflammations, des troubles ophtalmologiques, de la rétinopathie diabétique, de la dégénérescence maculaire, de la myopie, de la greffe de cornée, de l'histoplasmose oculaire, de la polyarthrite rhumatoïde, de l'arthrose, du glaucome rubéotique, de la rectocolite hémorragique, de la maladie de Crohn, de l'athérosclérose, du psoriasis, de la resténose, en particulier après angioplastie, de la sclérose en plaques, de la grossesse, de l'*absumptio placentaris,* des infections virales, des infections bactériennes, des infections fongiques, de la fièvre aphteuse (FMDV), du VIH, du charbon, du *candida albicans*, dans le cas d'une infestation parasitaire, dans le cas d'une insuffisance rénale aiguë et dans le cas de la guérison de plaies afin de favoriser le processus de guérison.
